(19) Europäisches Patentamt
European Patent Office
Office européen des brevets

(11)  **EP 4 065 959 B1**

(12)  **FASCICULE DE BREVET EUROPEEN**

(45) Date de publication et mention
de la délivrance du brevet:
**08.11.2023   Bulletin 2023/45**

(21) Numéro de dépôt: **20810975.1**

(22) Date de dépôt: **25.11.2020**

(51) Classification Internationale des Brevets (IPC):
**G01N 21/27** *(2006.01)*      **G01N 21/552** *(2014.01)*
**G01N 33/00** *(2006.01)*

(52) Classification Coopérative des Brevets (CPC):
**G01N 21/553;** G01N 33/0006; G01N 2201/0461;
G01N 2201/12761

(86) Numéro de dépôt international:
**PCT/EP2020/083372**

(87) Numéro de publication internationale:
**WO 2021/105213 (03.06.2021 Gazette 2021/22)**

(54) **PROCEDE DE CARACTERISATION DE COMPOSES D'INTÉRÊT DANS UNE CHAMBRE DE MESURE PRÉSENTANT UNE VARIATION D'HUMIDITÉ RELATIVE**

VERFAHREN ZUM CHARAKTERISIEREN VERBINDUNGEN IN EINER MESSKAMMER, DIE EINE SCHWANKUNG DER RELATIVEN FEUCHTIGKEIT AUFWEIST

METHOD FOR CHARACTERISING COMPOUNDS OF INTEREST IN A MEASURING CHAMBER HAVING A VARIATION IN RELATIVE HUMIDITY

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité:  **29.11.2019   FR 1913555**

(43) Date de publication de la demande:
**05.10.2022   Bulletin 2022/40**

(73) Titulaire: **Aryballe
38000 Grenoble (FR)**

(72) Inventeurs:
• **HERRIER, Cyril
38000 GRENOBLE (FR)**
• **HARBINE, David
38000 GRENOBLE (FR)**
• **CARITU, Yanis
38000 GRENOBLE (FR)**

(74) Mandataire: **Ipside
6, Impasse Michel Labrousse
31100 Toulouse (FR)**

(56) Documents cités:
**FR-A1- 2 716 975       JP-A- 2016 090 257
JP-A- 2016 138 782**

• **BRENET ET AL.: "Highly-Selective
Optoelectronic Nose based on Surface Plasmon
Resonance Imaging for Sensing Gas Phase
Volatile Organic Compounds", ANAL. CHEM.,
vol. 90, no. 16, 2018, pages 9879-9887,
XP002800118, cité dans la demande**
• **SHAO ET AL.: "Mechanism and Characteristics
of Humidity Sensing with Polyvinyl
Alcohol-Coated Fiber Surface Plasmon
Resonance Sensor", SENSORS, vol. 18, 2018,
page 2029, XP002800119, cité dans la demande**

**EP 4 065 959 B1**

# Description

## DOMAINE TECHNIQUE

Le domaine de l'invention est celui de la caractérisation de composés d'intérêt d'un échantillon gazeux situé dans une chambre de mesure présentant une variation d'humidité relative.

## ÉTAT DE LA TECHNIQUE ANTÉRIEURE

La capacité de caractériser et d'analyser des composés d'intérêt contenus dans des échantillons gazeux, par exemple des molécules odorantes ou des composés organiques volatils, est une problématique de plus en plus importante dans différents domaines, notamment dans ceux de la santé, de l'industrie agroalimentaire, de l'industrie de la parfumerie (senteurs), du confort olfactif dans les endroits confinés publics ou privés (automobile, hôtellerie, lieux partagés...), etc... La caractérisation des composés d'intérêt présents dans un échantillon gazeux est effectuée par un système de caractérisation appelé « nez électronique ».

Différentes approches de caractérisation existent, qui se distinguent entre elles notamment par la nécessité ou non d'avoir à « marquer » au préalable les composés d'intérêt ou les récepteurs par un agent de révélation. A la différence par exemple de la détection par fluorescence qui nécessite d'avoir recours à de tels marqueurs, la détection par résonance plasmonique de surface (SPR pour *Surface Plasmon Résonance,* en anglais) est une technique dite sans marqueur (*label free,* en anglais).

La technique de caractérisation SPR peut être mise en oeuvre par un nez électronique, utilisant par exemple la technologie d'imagerie SPR, les composés d'intérêt étant alors contenus dans un échantillon gazeux et venant interagir par adsorption/désorption avec des récepteurs situés dans une pluralité de sites sensibles distincts. Cette technique de caractérisation consiste à détecter en temps réel un signal optique, associé à chacun des sites sensibles, représentatif de la variation temporelle de l'indice de réfraction local du fait des interactions d'adsorption/désorption des composés d'intérêt avec les récepteurs.

Dans la mesure où on ne connaît pas *a priori* l'affinité chimique ou physique d'interaction des composés d'intérêt avec les récepteurs, la caractérisation des composés d'intérêt revient alors à déterminer une valeur d'équilibre (stationnaire) d'un paramètre représentatif des interactions d'adsorption/désorption des composés d'intérêt avec les récepteurs, ici représentatif de la variation temporelle de l'indice de réfraction local pour chacun des sites sensibles. On obtient ainsi un motif d'interaction, ou une signature, qui caractérise les composés d'intérêt. En effet, les interactions d'adsorption/désorption des composés d'intérêt sur des sites sensibles (surfaces fonctionnalisées) bénéficiant de caractéristiques d'adsorption différenciées permettent de rendre compte des molécules présentes dans le gaz qui ont été accrochées à la surface des différents sites sensibles.

A ce titre, les figures 1A et 1B illustrent un exemple de nez électronique tel que décrit dans la demande WO2018/158458. Ce type de nez électronique 1 comporte, d'une manière générale, un dispositif d'alimentation fluidique 2, un dispositif de mesure 3 par imagerie SPR, et une unité de traitement (non représentée).

Le dispositif de mesure 3 comporte une chambre de mesure 4 destinée à recevoir l'échantillon gazeux, dans laquelle se situe un support de mesure 5 sur lequel est située une matrice de sites sensibles $6_k$. Le support de mesure 5 est formé d'une couche métallique sur laquelle sont fixés différents récepteurs adaptés à interagir avec les composés d'intérêt, les différents récepteurs étant disposés de manière à former des sites sensibles $6_k$ distincts les uns des autres. Ces récepteurs sont alors situés à l'interface entre la couche métallique et un milieu diélectrique, ici un milieu gazeux.

Ce dispositif de mesure 3 comporte en outre une source lumineuse 7 d'un signal optique d'excitation et un capteur d'image 8. Au moins une lentille de focalisation ou collimation et au moins un polariseur peuvent être prévus sur le chemin optique entre la source 7 et le capteur 8, de manière connue. La source lumineuse 7 est adaptée à émettre le signal optique d'excitation en direction du support de mesure 5, suivant un angle de travail $\theta_R$ permettant d'y générer des plasmons de surface. La partie réfléchie du signal optique d'excitation, formant un signal optique de mesure, est ensuite détectée par le capteur d'image 8. L'intensité du signal optique de mesure dépend localement de l'indice de réfraction du support de mesure 5, qui dépend lui-même des plasmons de surface générés et de la quantité de matière située au niveau de chaque site sensible $6_k$, cette quantité de matière variant au cours du temps au grès des interactions entre les composés sensibles et les récepteurs.

L'unité de traitement du nez électronique est adaptée à analyser les « sensorgrammes », c'est-à-dire les signaux correspondant à l'évolution temporelle du paramètre représentatif des interactions d'adsorption/désorption des composés d'intérêt avec les récepteurs de chacun des différents sites sensibles $6_k$, dans le but d'en extraire les informations de la cinétique d'interaction (adsorption et désorption) des composés d'intérêt avec les récepteurs. Ces sensorgrammes peuvent être des signaux de mesure $S_k(t)$ correspondant à l'intensité du signal de mesure détecté en temps réel par le capteur d'image 8 de chacun des sites sensibles $6_k$, ou être des signaux dits utiles $Su_k(t)$ correspondant à l'évolution temporelle de la variation $\Delta\%R_k(t)$ de la réflectivité associée à chacun des sites sensibles $6_k$. La réflectivité $\%R$ est définie comme le rapport entre l'intensité du signal optique de mesure détecté par le capteur 8 sur l'intensité du signal optique d'excitation émis par la source 7. La variation de réflectivité $\Delta\%R$ est obtenue en soustrayant à l'évolution temporelle de la réflectivité %R(t) une valeur de référence (*baseline,* en anglais) associée au gaz seul présent à l'intérieur de la chambre de mesure, indépendamment des composés d'intérêt.

Aussi, les signaux utiles $Su_k(t)$ présentent une même valeur initiale stationnaire, de préférence sensiblement égale à zéro, avant l'introduction des composés d'intérêt dans la chambre de mesure 4. Ainsi, cette valeur de référence (*baseline,* en anglais) qui traduit l'impact du seul gaz (sans les composés d'intérêt) sur chacun des sites sensibles $6_k$ est soustraite au signal de mesure $S_k(t)$ correspondant. L'intensité des signaux utiles $Su_k(t)$ témoigne alors d'un ajout des composés d'intérêt dans la chambre de mesure 4.

Enfin, le dispositif d'alimentation fluidique 2 est adapté à introduire les composés d'intérêt dans la chambre de mesure 4 dans des conditions permettant l'analyse des sensorgrammes et donc la caractérisation des composés d'intérêt. A ce titre, l'article de Brenet et al. intitulé Highly-Selective Optoelectronic Nose based on Surface Plasmon résonance Imaging for Sensing Gas Phase Volatile Organic Compounds, Anal. Chem. 2018, 90, 16, 9879-9887, décrit un procédé de caractérisation d'un échantillon gazeux au moyen d'un nez électronique de type à imagerie SPR.

Le procédé de caractérisation consiste à alimenter la chambre de mesure en un échantillon gazeux de telle manière que la cinétique d'interaction entre les composés d'intérêt et les récepteurs atteigne un régime stationnaire d'équilibre. Plus précisément, l'étape d'injection fluidique comporte successivement :

- une première phase P1 dite initiale, dans laquelle un gaz de référence seul, sans les composés d'intérêt, est injecté dans la chambre de mesure. Ce gaz de référence est généralement identique au gaz porteur de l'échantillon gazeux ;
- une deuxième phase P2 dite de caractérisation, dans laquelle l'échantillon gazeux, formé du gaz porteur et des composés d'intérêt, est injecté dans la chambre de mesure ;
- une troisième phase P3 dite de dissociation, dans laquelle les composés d'intérêt sont évacués de la chambre de mesure.

La phase initiale P1 permet d'acquérir la valeur de référence (*baseline*) mentionnée plus haut destinée à être ensuite soustraite aux signaux de mesure $S_k(t)$ pour obtenir des signaux utiles $Su_k(t)$ (autrement dit l'évolution temporelle de la variation de réflectivité $\Delta\%R_k(t)$ pour chaque site sensible). Comme indiqué précédemment, cette étape d'injection fluidique est effectuée de sorte que les sensorgrammes mettent en évidence la présence d'un régime transitoire d'assimilation suivi d'un régime stationnaire d'équilibre. Lorsque ce régime stationnaire d'équilibre est atteint, les valeurs d'équilibre (stationnaires) des signaux utiles $Su_k(t)$ sont extraites par l'unité de traitement, et définissent la signature des composés d'intérêt.

Or, il apparaît que l'humidité relative à l'intérieur de la chambre de mesure a un impact sur l'intensité du signal optique de mesure, comme l'indique l'article de Shao et al. intitulé Mechanism and Characteristics ofhumidity Sensing with Polyvinyl Alcohol-Coated Fiber Surface Plasmon résonance Sensor, Sensors 2018, 18, 2029. Dans cet article, les auteurs utilisent un capteur SPR comme d'un capteur d'humidité. Cependant, dans le cadre d'un procédé de caractérisation des composés d'intérêt par un nez électronique, la variation d'humidité relative dans la chambre de mesure forme un bruit de mesure dégradant la qualité de la caractérisation. De plus, dans le cas où l'humidité relative varie sur des temps longs, et donc varie d'une caractérisation à l'autre pour les mêmes composés d'intérêt et les mêmes conditions opératoires, cela induit une dérive temporelle qui rend les signatures de ces mêmes composés d'intérêt différentes les unes des autres.

## EXPOSÉ DE L'INVENTION

L'invention a pour objectif de remédier au moins en partie aux inconvénients de l'art antérieur, et plus particulièrement de proposer un procédé de caractérisation de composés d'intérêt qui limite voire écarte le bruit de mesure lié à la différence d'humidité relative dans la chambre de mesure entre la phase initiale P1 et la phase de caractérisation P2.

Pour cela, l'objet de l'invention est un procédé selon la revendication 1 de caractérisation de composés d'intérêt introduits dans une chambre de mesure d'un nez électronique comportant au moins un site sensible ayant des récepteurs avec lesquels les composés d'intérêt sont aptes à interagir par adsorption/désorption, le procédé comportant les étapes suivantes :

- injection, dans la chambre de mesure : pendant une première phase P1, d'un premier échantillon gazeux formé d'un gaz porteur sans les composés d'intérêt, puis, pendant une deuxième phase P2, d'un deuxième échantillon gazeux formé d'au moins le gaz porteur et desdits composés d'intérêt ;
- détermination, au cours desdites phases P1 et P2, d'un signal de mesure représentatif des interactions entre l'échantillon gazeux présent et les récepteurs du site sensible $6_k$, à différents instants de mesure $t_i$, en réponse à un signal d'excitation émis au niveau du site sensible.

Selon l'invention, le procédé comporte les étapes suivantes :

- mesure d'une valeur $\varphi 1$ de l'humidité relative $\varphi$ dans la chambre de mesure lors de la première phase P1, et d'une valeur $\varphi 2$ lors de la deuxième phase P2, $\varphi 2$ étant différente de $\varphi 1$ ;
- détermination d'un paramètre correctif associé au

site sensible ($6_k$), à partir : d'au moins la valeur mesurée φ2 de l'humidité relative ; et d'une fonction de calibration prédéterminée et associée au site sensible, la fonction de calibration exprimant une variation d'un paramètre représentatif du signal de mesure associé au premier échantillon gazeux en fonction de l'humidité relative ;

- détermination d'un signal utile par correction du signal de mesure associé au deuxième échantillon gazeux à partir d'au moins le paramètre correctif déterminé, et caractérisation des composés d'intérêt à partir du signal utile.

Certains aspects préférés mais non limitatifs de ce procédé de caractérisation sont les suivants.

Le procédé peut comporter une phase de détermination de la fonction de calibration notée $h_k$ comportant les étapes suivantes :

- injection du premier échantillon gazeux dans la chambre de mesure de sorte que l'humidité relative φ varie progressivement, et mesure de l'humidité relative φ ;
- détermination d'un signal de mesure $\tilde{S}_k(t_i)$ au cours de l'étape d'injection, puis détermination d'une valeur de référence $\tilde{S}_k^{ref}$ à partir du signal de mesure $\tilde{S}_k(t_i)$ déterminé et pour chaque valeur d'humidité relative φ mesurée ;
- détermination d'une fonction de calibration $h_k$ exprimant la variation de la valeur de référence $\tilde{S}_k^{ref}$ en fonction de l'humidité relative φ, à partir des valeurs de référence $\tilde{S}_k^{ref}$ déterminées et des valeurs d'humidité relative φ mesurées.

Le paramètre correctif peut être une valeur de référence $\tilde{S}_k^{ref}\big|_{φ2}$ représentative du signal de mesure $S_k(t_i)$ du premier échantillon gazeux pour l'humidité relative φ2 mesuré.

L'étape de détermination du signal utile peut comporter les sous-étapes suivantes :

- détermination d'une valeur de référence $\tilde{S}_k^{ref}\big|_{φ1}$ représentative du signal de mesure du premier échantillon gazeux pour l'humidité relative φ1 mesuré, à partir de la fonction de calibration $h_k$ ;
- détermination d'un signal utile $Su_k(t_i \in P1)$ associé au premier échantillon par soustraction au signal de mesure $S_k(t \in P1)$ déterminé lors de la phase initiale P1 de la valeur de référence $\tilde{S}_k^{ref}\big|_{φ1}$ déterminée ;
- détermination d'une valeur de référence $Su_k^{ref}$ à partir du signal utile déterminé $Su_k(t_i \in P1)$ associé au premier échantillon ;
- détermination d'un signal utile corrigé $Suc_k(t_i \in P2)$ associé au deuxième échantillon gazeux par soustraction au signal utile $Su_k(t_i \in P2)$ de la valeur de référence $Su_k^{ref}$ déterminée. La caractérisation (150) des composés d'intérêt peut être effectuée à partir du signal utile corrigé $Suc_k(t_i \in P2)$ associé au deuxième échantillon gazeux.

Le procédé peut comporter une phase de détermination de la fonction de calibration notée $f_k$ comportant les étapes suivantes :

- injection du premier échantillon gazeux dans la chambre de mesure de sorte que plusieurs cycles d'injection soient effectués, chaque cycle comportant une première injection du premier échantillon gazeux à une humidité relative φ1 suivie d'une deuxième injection du premier échantillon gazeux à une humidité relative φ2 différente de φ1, et détermination de la différence d'humidité relative Δφ entre φ1 et φ2 variant d'un cycle à l'autre ;
- détermination d'un signal de mesure $\tilde{S}_k(t_i)$ au cours des différents cycles d'injection, et détermination d'une différence de valeurs de référence $\Delta\tilde{S}_k^{ref}$ pour chaque cycle d'injection et pour chaque différence d'humidité relative Δφ déterminée, à partir du signal de mesure $\tilde{S}_k(t_i)$ déterminé au cours des différents cycles d'injection ;
- détermination de la fonction de calibration $f_k$ exprimant la variation de la différence de valeur de référence $\Delta\tilde{S}_k^{ref}$ en fonction de la différence d'humidité relative Δφ, à partir des valeurs déterminées de la différence de valeur de référence $\Delta\tilde{S}_k^{ref}$ et des valeurs déterminées de la différence d'humidité relative Δφ.

Le paramètre correctif peut être la somme d'une valeur de la différence de valeurs de référence $\Delta\tilde{S}_k^{ref}$ pour la différence d'humidité relative Δφ déterminée à partir de l'humidité relative mesurée lors de la première phase P1 et de la deuxième phase P2 ; et d'une valeur de référence $S_k^{ref}\big|_{φ1}$ associée au premier échantillon gazeux et déterminée à partir du signal de mesure $S_k(t_i \in P1)$ déterminé lors de la première phase P1.

L'étape de détermination du signal utile peut comporter la soustraction du paramètre correctif au signal de mesure $S_k(t_i \in P2)$ associé au deuxième échantillon gazeux et déterminé lors de la deuxième phase P2.

La fonction de calibration peut être une fonction polynomiale, logarithmique, ou exponentielle. Elle peut être une fonction polynomiale d'ordre 2.

Le nez électronique peut comporter un dispositif de mesure des interactions entre les composés d'intérêt et les récepteurs de type optique à résonance des plasmons de surface. En variante, il peut comporter un dispositif de mesure des interactions entre les composés d'intérêt et les récepteurs de type résistif, piézoélectrique, mécanique, acoustique ou optique.

**BRÈVE DESCRIPTION DES DESSINS**

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :

les figures 1A et 1B, déjà décrites, sont des vues schématiques et partielles, en coupe (fig.1A) et en vue de dessus (fig.1B), d'un nez électronique à imagerie SPR selon un exemple de l'art antérieur et des sites sensibles du support de mesure ;

la figure 1C est une vue schématique et partielle d'un nez électronique à imagerie SPR selon un mode de réalisation ;

la figure 2 est un exemple de sensorgrammes $Su_k(t)$ mesurés par le nez électronique selon l'exemple de l'art antérieur, ces sensorgrammes correspondant ici à l'évolution temporelle de la variation de la réflectivité $\Delta\%R_k(t)$ associée à chacun des sites sensibles $6_k$;

la figure 3A est un exemple de trois motifs d'interaction (signatures) obtenus par un procédé de caractérisation selon l'art antérieur, mettant en évidence la dégradation de la caractérisation de composés d'intérêt du fait d'une différence d'humidité relative dans la chambre de mesure entre la phase initiale P1 et la phase de caractérisation P2 ;

la figure 3B est un exemple de différents signaux de mesure $S_k(t)$ obtenus par un nez électronique à imagerie SPR pour différentes situations d'humidité relative $\varphi$ dans la chambre de mesure : i.e. dans le cas où l'humidité relative est constante et égale à $\varphi 1$, dans le cas où l'humidité relative est constante et égale à $\varphi 2$ différente de $\varphi 1$, et dans le cas où l'humidité relative passe de $\varphi 1$ à $\varphi 2$ entre la phase initiale P1 et la phase de caractérisation P2 ;

la figure 4 est un organigramme d'un procédé de caractérisation selon un premier mode de réalisation ;

la figure 5 est un exemple d'une fonction de calibration $h_k$ exprimant l'évolution d'une valeur de référence $\tilde{S}_k^{ref}$ associée à un premier échantillon gazeux (gaz porteur sans les composés d'intérêt) en fonction de l'humidité relative $\varphi$ ;

la figure 6 illustre les trois motifs d'interaction (signatures) déjà illustrés sur la fig.3A, ainsi qu'un motif d'interaction obtenu par le procédé de caractérisation selon le premier mode de réalisation ;

la figure 7 est un organigramme d'un procédé de caractérisation selon un deuxième mode de réalisation ;

la figure 8A est un exemple d'une différence $\Delta S_k(t)$ entre des signaux de mesure $S_k(t)$ pour différents cycles d'injection d'un premier échantillon gazeux (gaz porteur sans les composés d'intérêt), chaque cycle comportant une injection du premier échantillon gazeux d'humidité relative $\varphi 1$ constante au cours des cycles, et d'une injection du même premier échantillon gazeux d'humidité relative $\varphi 2$ qui varie au cours des cycles ;

la figure 8B illustre la différence d'humidité relative $\Delta\varphi$ au cours des cycles illustrés sur la fig.8A ;

la figure 8C est un exemple d'une fonction de calibration $h_k$ exprimant l'évolution de la différence de valeur de référence $\Delta\tilde{S}_k^{ref}$ en fonction de la différence d'humidité relative $\Delta\varphi$.

**EXPOSÉ DÉTAILLÉ DE MODES DE RÉALISATION PARTICULIERS**

Sur les figures et dans la suite de la description, les mêmes références représentent les éléments identiques ou similaires. De plus, les différents éléments ne sont pas représentés à l'échelle de manière à privilégier la clarté des figures. Par ailleurs, les différents modes de réalisation et variantes ne sont pas exclusifs les uns des autres et peuvent être combinés entre eux. Sauf indication contraire, les termes « sensiblement », « environ », « de l'ordre de » signifient à 10% près, et de préférence à 5% près. Par ailleurs, les termes « compris entre ... et ... » et équivalents signifient que les bornes sont incluses, sauf mention contraire.

L'invention porte sur la caractérisation de composés d'intérêt présents dans un gaz porteur formant un échantillon gazeux à analyser. La caractérisation est effectuée au moyen d'un système d'analyse appelé 'nez électronique', lequel comporte : un dispositif de mesure ; un dispositif d'alimentation fluidique ; un capteur d'humidité ; et une unité de traitement. Comme détaillé plus loin, le dispositif de mesure comporte une chambre de mesure adaptée à recevoir l'échantillon gazeux, dans laquelle est située au moins un site sensible, et de préférence une pluralité de sites sensibles distincts, le ou les sites sensibles ayant chacun des récepteurs adaptés à interagir par adsorption/désorption avec les composés d'intérêt.

A titre d'illustration, le nez électronique utilise la technologie de mesure par résonance plasmonique de surface (SFR). Le dispositif de mesure comporte alors un capteur optique qui peut être un capteur d'image, la chambre de mesure ayant alors une pluralité de sites sensibles $6_k$ (k étant le rang du site sensible considéré), ou qui peut être un photodétecteur, auquel cas, la mesure est réalisée par la recherche de l'angle de réflectivité minimal, cet angle étant représentatif de la variation d'indice. En variante, d'autres technologies de mesure peuvent être mises en oeuvre, telles que la mesure par résonateurs électromagnétiques de type MEMS ou NEMS. Plus largement, le dispositif de mesure peut être de type résistif, piézoélectrique, mécanique, acoustique ou optique.

D'une manière générale, par caractérisation on entend l'obtention d'informations représentatives des interactions des composés d'intérêt contenus dans l'échantillon gazeux avec les récepteurs du ou des sites sensibles du nez électronique. Les interactions en question ici sont des évènements d'adsorption et/ou de désorption des composés d'intérêt avec les récepteurs. Ces informations forment ainsi un motif d'interaction, autrement dit une « signature » des composés d'intérêt, ce motif pouvant être représenté par exemple sous forme d'histogramme ou d'un diagramme en radar. Plus précisément, dans le cas où le nez électronique comporte N sites sensibles distincts, le motif d'interaction est formé par les N informations représentatives, celles-ci étant issues du signal de mesure associé au site sensible $6_k$ considéré.

D'une manière générale, les composés d'intérêt (*analytes,* en anglais) sont des éléments destinés à être caractérisés par le nez électronique, et contenus dans un échantillon gazeux. Ils peuvent être, à titre illustratif, des bactéries, virus, protéines, lipides, molécules organiques volatiles, composés inorganiques, entre autres. Par ailleurs, les récepteurs (*ligants,* en anglais) sont des éléments fixés aux sites sensibles et qui présentent une capacité d'interaction avec les composés d'intérêt, bien que les affinités chimique et/ou physique entre les composés sensibles et les récepteurs ne soient pas nécessairement connues. Les récepteurs des différents sites sensibles présentent de préférence des propriétés physico-chimiques différentes, qui impactent leur capacité à interagir avec les composés d'intérêt. Il peut s'agir, à titre d'exemples, des acides aminés, des peptides, des nucléotides, des polypeptides, des protéines, des polymères organiques, entre autres.

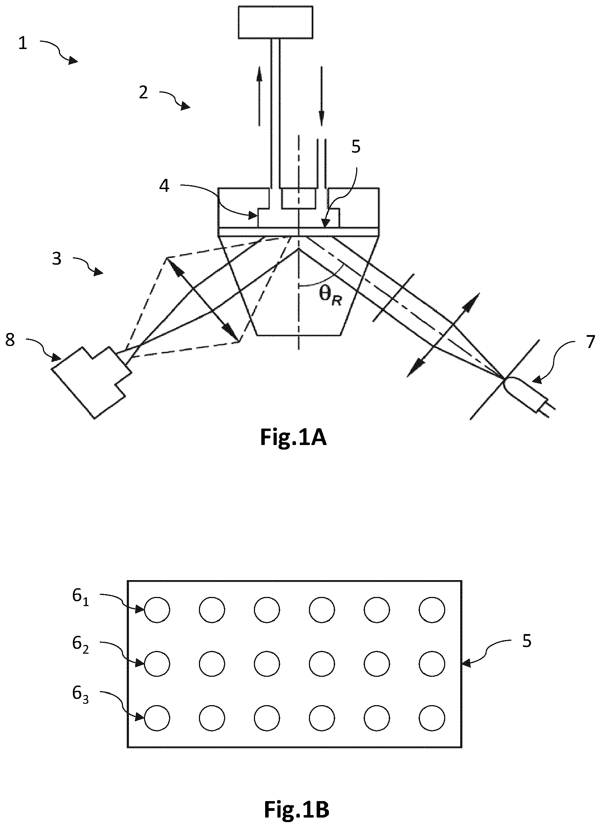

En référence aux fig.1A à 1C, le nez électronique 1 est ici un système optoélectronique permettant de caractériser des composés d'intérêt, par exemple des molécules odorantes, des composés organiques volatils ou autres, contenus dans un échantillon gazeux introduit dans une chambre de mesure 4 du nez électronique. Le nez électronique 1 représenté sur ces figures repose ici sur la technologie SPR et présente dans cet exemple les caractéristiques de la configuration dite de Kretschmann, connue de l'homme du métier, sans que l'invention ne soit toutefois limitée à cette configuration. Toutefois, comme indiqué précédemment, d'autres techniques de mesure peuvent être utilisées, comme les mesures de la fréquence de résonance d'un microrésonateur de type MEMS ou NEMS fonctionnalisé de sorte qu'il présente au moins un site sensible muni de récepteurs.

Le nez électronique 1 comporte une pluralité de sites sensibles $6_k$ distincts les uns des autres et situés dans une chambre de mesure 4 destinée à recevoir l'échantillon gazeux à analyser, ces sites sensibles étant formés chacun des récepteurs aptes à interagir avec les composés d'intérêt à étudier (cf. fig.1B). Les sites sensibles $6_k$ sont distincts les uns des autres, dans le sens où ils comportent des récepteurs différents, en termes d'affinité chimique ou physique vis-à-vis des composés d'intérêt à analyser, et sont donc destinés à fournir une information d'interaction différente d'un site sensible $6_k$ à l'autre. Les sites sensibles $6_k$ sont des zones distinctes d'un support de mesure 5, et peuvent être accolés ou espacés les unes des autres. Le nez électronique 1 peut en outre comporter plusieurs sites sensibles $6_k$ identiques, dans le but par exemple de détecter une éventuelle dérive de mesure et/ou de permettre l'identification d'un site sensible défectueux.

Le nez électronique comporte un dispositif de mesure 3, ici de type imagerie SPR, permettant de quantifier les interactions des composés d'intérêt avec les récepteurs, pour chaque site sensible $6_k$, ici en mesurant en temps réel l'intensité d'un signal optique de mesure provenant du site sensible $6_k$ considéré, ce signal optique étant ici une partie réfléchie d'un signal optique d'excitation émis par une source lumineuse 7. L'intensité du signal optique de mesure détecté par le capteur optique 8 est directement corrélée notamment aux interactions d'adsorption/désorption des composés d'intérêt avec les récepteurs. Dans le cas des techniques de mesure de la fréquence de résonance d'un microrésonateur NEMS ou MEMS, le signal de mesure peut être un signal électrique représentatif de la vibration d'une micropoutre ou équivalent.

Dans le cadre d'une mesure par imagerie SPR, le dispositif de mesure 3 est adapté à acquérir en temps réel le signal optique de mesure provenant de l'ensemble des sites sensibles $6_k$. Ainsi, les signaux optiques de mesure provenant des sites sensibles $6_k$ en réponse au signal optique d'excitation sont détectés ensemble et en temps réel, sous la forme d'une image acquise par le même capteur optique 8.

Ainsi, le dispositif de mesure optique 3 comporte une source lumineuse 7 adaptée à transmettre un signal optique dit d'excitation en direction des sites sensibles $6_k$, et à générer des plasmons de surface au niveau du support de mesure 5. La source lumineuse 7 peut être formée d'une diode électroluminescente, dont le spectre d'émission présente un pic d'émission centré sur une longueur d'onde centrale $\lambda_c$. Différents éléments optiques (lentilles, polariseur...) peuvent être disposés entre la source lumineuse 7 et le support de mesure 5.

Le dispositif de mesure optique 3 comporte en outre un capteur optique 8, et ici un capteur d'image, c'est-à-dire un capteur optique matriciel adapté à collecter ou détecter une image du signal optique provenant des sites sensibles en réponse au signal optique d'excitation. Le capteur d'image 8 est un photodétecteur matriciel, par exemple un capteur CMOS ou CCD. Il comporte donc une matrice de pixels dont la résolution spatiale est telle que, de préférence, plusieurs pixels acquièrent le signal optique de mesure provenant d'un même site sensible $6_k$.

L'unité de traitement (non représentée) permet la mise en oeuvre des opérations de traitement décrites par la suite dans le cadre du procédé de caractérisation. Elle peut comporter au moins un microprocesseur et au moins une mémoire. Elle est connectée au dispositif de mesure optique 3, et plus précisément au capteur d'image 8. Elle comporte un processeur programmable apte à exécuter des instructions enregistrées sur un support d'enregistrement d'informations. Elle comporte en outre au moins une mémoire contenant les instructions nécessaires à la mise en oeuvre du procédé de caractérisation. La mémoire est également adaptée à stocker les informations calculées à chaque instant de mesure.

Comme décrit plus loin, l'unité de traitement est notamment adaptée à stocker et à traiter une pluralité d'images dites élémentaires acquises à une fréquence d'échantillonnage $f_e$ donnée, sur une durée de mesure $\Delta t$, afin de déterminer un signal de mesure $S_k(t_i)$, à l'instant courant $t_i$, associé au site sensible $6_k$. De préférence, le signal de mesure $S_k(t_i)$ correspond, à un instant de mesure $t_i$, à la moyenne de l'intensité du signal optique réfléchi et détecté par le capteur d'image 8 sur les pixels associés au site sensible $6_k$. La moyenne de l'intensité optique détectée sur les pixels peut être effectuée pour une ou plusieurs images du site sensible $6_k$, comme décrit en détail plus loin.

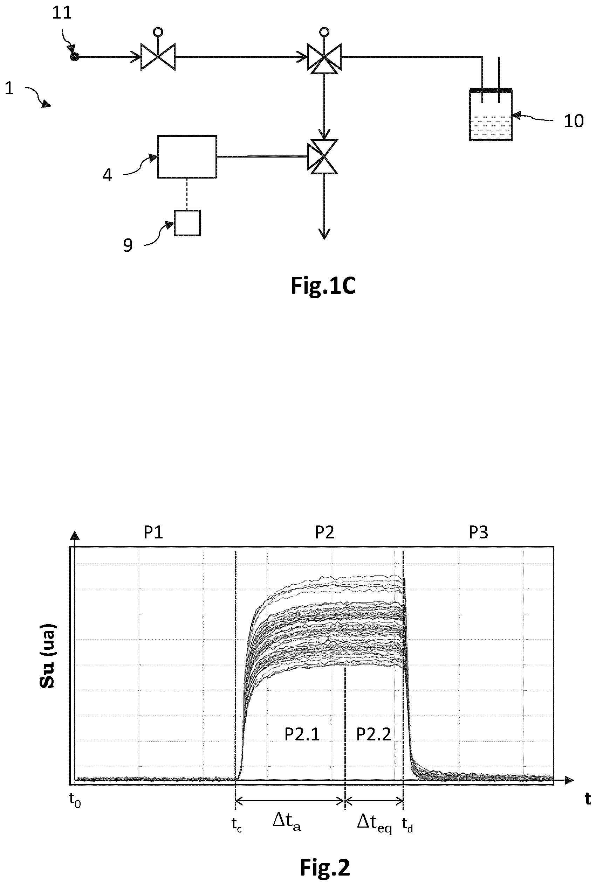

Le dispositif d'alimentation fluidique 2 est adapté à alimenter la chambre de mesure 4 en un premier échantillon gazeux formé du gaz porteur seul (i.e. sans les composés d'intérêt) pendant la phase initiale P1, et en un deuxième échantillon gazeux formé du gaz porteur et des composés d'intérêt pendant la phase de caractérisation P2. Le deuxième échantillon gazeux diffère du premier échantillon essentiellement en ce qu'il comporte les composés d'intérêt. Un ou plusieurs gaz additionnels peuvent être présents, mais sont inodores de sorte qu'ils n'induisent sensiblement pas de réponse de la part du nez électronique 1. Un exemple de gaz additionnel présent dans le deuxième échantillon gazeux peut être le diluant en phase vapeur. Comme décrit en référence à la fig.1C, les composés d'intérêt peuvent être stockés dans un diluant liquide contenu dans un réservoir 10. La phase vapeur du diluant et les composés d'intérêt sont ajoutés au gaz porteur (par ex. de l'air humide) pour former le deuxième échantillon gazeux. Les premier et deuxième échantillons gazeux diffèrent l'un de l'autre ici également par leur valeur d'humidité relative.

Pour cela, comme l'illustre la fig.1C, le dispositif d'alimentation fluidique 2 comporte une entrée 11 de gaz porteur, et un réservoir 10 de composés d'intérêt. Ici, le réservoir 10 contient un diluant dans lequel se situent les composés d'intérêt. Il comporte plusieurs lignes fluidiques qui relient l'entrée 11 du gaz porteur et le réservoir 10 d'une part, à l'entrée de la chambre de mesure 4 d'autre part, et comporte des vannes et éventuellement des régulateurs d'écoulement massique. Il permet ainsi d'alimenter la chambre de mesure 4 en le premier échantillon gazeux (par ex. air humide à l'humidité relative $\varphi1$) lors de la phase initiale P1 et de la phase de dissociation P3, et en le deuxième échantillon gazeux (par ex. air humide à l'humidité relative $\varphi2$, composés d'intérêt, et diluant en phase vapeur) lors de la phase de caractérisation P2. Il peut être adapté à assurer que la concentration des composés d'intérêt dans la chambre de mesure reste constante au cours du temps. Par ailleurs, le nez électronique selon un mode de réalisation comporte en outre un capteur d'humidité 9 apte à mesurer l'humidité relative dans la chambre de mesure. Le capteur d'humidité 9 peut être disposé dans la chambre de mesure, ou en amont ou en aval de celle-ci. Il est connecté à l'unité de traitement, qui peut en outre être adaptée à calculer une différence d'humidité relative entre la phase initiale P1 et la phase de caractérisation P2.

La figure 2 illustre un exemple de sensorgrammes $Su_k(t)$ associés à des sites sensibles $6_k$ d'un nez électronique à imagerie SPR, dans le cadre d'un procédé de caractérisation dans lequel les sensorgrammes présentent chacun un profil dit conventionnel, c'est-à-dire qu'ils mettent en évidence la présence d'un régime d'équilibre (i.e. stationnaire) d'interactions entre les composés d'intérêt et les récepteurs. Dans cet exemple, l'humidité relative $\varphi$ ne varie sensiblement pas entre la phase initiale P1 et la phase de caractérisation P2.

Un sensorgramme $Su_k(t)$ correspond à l'évolution temporelle d'un paramètre de caractérisation représentatif des interactions entre les composés d'intérêt et les récepteurs d'un site sensible considéré $6_k$. Il est déterminé à partir de l'intensité du signal de mesure $S_k(t)$ provenant du site sensible $6_k$ en réponse à l'émission d'un signal d'excitation. Dans cet exemple, le paramètre de caractérisation est la variation $\Delta\%R_k(t)$ par rapport à une valeur de référence (*baseline,* en anglais) de la réflectivité $\%R_k(t)$ associée au site sensible $6_k$, mais il peut s'agir, dans une autre configuration du nez électronique, de la variation du coefficient de transmission. La variation de la réflectivité $\Delta\%R_k(t)$ est ici corrélée à la modification de l'indice de réfraction du site sensible $6_k$ considéré, laquelle dépend des interactions d'adsorption et de désorption des composés d'intérêt avec les récepteurs du site sensible $6_k$.

De manière connue, un sensorgramme $Su_k(t)$

à profil conventionnel présente une phase initiale P1, une phase de caractérisation des composés d'intérêt P2, puis une phase de dissociation P3. La valeur en ordonnée du sensorgramme $Su_k(t)$ est notamment proportionnelle au nombre de récepteurs du site sensible $6_k$ considéré.

La phase initiale P1 correspond à l'introduction dans la chambre de mesure, à partir de l'instant $t_o$ et jusqu'à l'instant $t_c$, du premier échantillon (gaz porteur ne contenant pas les composés d'intérêt). Les signaux de mesure $S_k(t)$, autrement dit l'évolution temporelle de la réflectivité %R(t) déterminée pour chaque site sensible $6_k$ entre $t_o \leq t < t_c$, caractérisent l'environnement dans la chambre de mesure pour chacun des sites sensibles $6_k$. On en déduit ensuite une valeur de référence $\tilde{S}_k^{ref}$ (*base-line*) généralement différente d'un site sensible $6_k$ à l'autre, qui est ensuite soustraite au signal de mesure $S_k(t)$ pour obtenir le signal utile $Su_k(t)$. Ainsi, les sensorgrammes illustrent les signaux utiles $Su_k(t)$, lesquels présentent donc, lors de la phase initiale P1, une même valeur initiale proche de zéro pour l'ensemble des sites sensibles $6_k$.

La phase d'injection P2 correspond à l'introduction dans la chambre de mesure, à partir de l'instant $t_c$ jusqu'à l'instant $t_d$, du deuxième échantillon gazeux (gaz porteur et composés d'intérêt, et éventuellement un diluant inodore en phase gazeuse). Cette phase comporte un régime transitoire d'assimilation P2.1 suivi d'un régime stationnaire d'équilibre P2.2. Dans ces exemples, l'humidité relative φ est constante au cours des différentes phases P1, P2 et P3.

Le régime transitoire d'assimilation P2.1 correspond à l'augmentation progressive, de forme exponentielle (loi approchée de Langmuir), des interactions entre les composés d'intérêt et les récepteurs, à mesure que les composés d'intérêt sont injectés dans la chambre de mesure. La croissance exponentielle des sensorgrammes dans le régime d'assimilation est due au fait qu'il y a alors bien plus d'évènements d'adsorption que d'évènements de désorption.

Notons à cet égard que l'interaction entre un composé d'intérêt A (*analyte* en anglais) et un récepteur L (*ligand,* en anglais) est un phénomène réversible caractérisé par une constante d'adsorption $k_a$ (en $mol^{-1}.s^{-1}$) du composé d'intérêt A au récepteur L pour former un composé d'intérêt/récepteur LA (pour *ligand-analyte,* en anglais), et par une constante de désorption $k_b$ (en $s^{-1}$) correspondant à la dissociation du composé LA. Le ratio $k_d/k_a$ forme la constante de dissociation d'équilibre $k_D$ (en mol) qui donne la valeur de la concentration $c_A$ de composés d'intérêt A permettant de saturer 50% des récepteurs L.

Le régime stationnaire d'équilibre P2.2 est atteint lorsque la concentration $c_{LA}(t)$ en composés LA est stationnaire $dc_{LA}/dt=0$, c'est-à-dire lorsque le produit de la constante $k_a$ avec les concentrations de composés d'intérêt $c_A(t)$ et de récepteurs $c_L(t)$ (nombre d'évènements d'adsorption) est égal au produit de la constante $k_d$ avec la concentration $c_{LA}(t)$ de composés LA (nombre d'évènements de désorption), autrement dit lorsque l'équation d'évolution suivante est vérifiée $dc_{LA}/dt = k_a \times c_A \times c_L - k_d \times c_{LA} = 0$. La valeur maximale stationnaire du signal de mesure est proportionnelle à la concentration $c_A(t)$ de composés d'intérêt A. La saturation des récepteurs L du site sensible peut être atteinte lorsque la concentration $c_A$ en composés d'intérêt A est suffisante.

La phase de dissociation P3 correspond à une étape d'évacuation des composés d'intérêt présents dans la chambre de mesure, à partir de l'instant $t_d$, de sorte que la concentration en composés LA diminue, habituellement de manière exponentielle. Il peut s'agir d'introduire à nouveau le premier échantillon gazeux dans la chambre de mesure.

Or, il apparaît que la variation d'humidité relative au sein de la chambre de mesure au cours de l'étape d'injection fluidique forme un bruit de mesure qui dégrade la qualité de la caractérisation. Ce bruit de mesure est un bruit lié à une différence non nulle de l'humidité relative au sein de la chambre de mesure entre la phase initiale P1 et la phase de caractérisation P2. Il s'agit d'un bruit de mesure dans la mesure où il est issu d'une variation temporelle d'un paramètre (ici l'humidité relative) qui caractérise l'environnement à l'intérieur de la chambre de mesure et devrait théoriquement rester stationnaire au cours du temps.

Autrement dit, l'humidité relative peut présenter une première valeur φ1 sensiblement constante au cours de la phase initiale P1, et une deuxième valeur φ2 sensiblement constante mais différente de la valeur φ1 au cours de la phase de caractérisation P2. On définit ici la différence d'humidité relative Δφ comme étant égale à φ2-φ1. Par humidité relative φ, on entend la teneur en vapeur d'eau du gaz présent dans la chambre de mesure, et ici du gaz porteur. Il s'agit du rapport entre la pression partielle de la vapeur d'eau contenue dans le gaz présent et la pression de vapeur saturante à la même température.

Ce bruit de mesure est présent notamment lorsque le deuxième échantillon gazeux présente une humidité relative φ2 différente, par exemple inférieure, à celle φ1 du premier échantillon gazeux. Ainsi, lors de la phase initiale P1, le premier échantillon gazeux peut être de l'air humide d'humidité relative φ1 issu de l'environnement du nez électronique. Et lors de la phase de caractérisation P2, le deuxième échantillon gazeux est formé d'air humide issu par exemple de l'environnement du nez électronique, ainsi que des composés d'intérêt issu du réservoir 10. Cependant, l'humidité relative φ2 du deuxième échantillon gazeux peut être différente de φ1. En effet, l'humidité relative de l'air humide provenant de l'environnement peut avoir changé. Une autre possibilité expliquant la variation d'humidité relative passant de φ1 à φ2 peut provenir de l'humidité relative du gaz présent dans l'espace de tête (*headspace,* en anglais) du réservoir 10. En effet, pour former le deuxième échantillon gazeux, de l'air humide à φ1 provenant de l'entrée 11 de gaz porteur est introduit dans le réservoir 10 et mélangé au gaz présent (diluant en phase gazeuse et composés d'intérêt). Or, le diluant en phase liquide peut être hydrophile, de sorte qu'il peut alors conduire à une diminution de l'humidité relative $\varphi$ de l'air humide introduit dans l'espace de tête du réservoir 10. En conséquence, le deuxième échantillon gazeux va présenter une humidité relative $\varphi2$ inférieure à $\varphi1$.

Notons qu'un équilibre thermodynamique peut progressivement s'établir dans l'espace de tête du réservoir 10, de sorte que le diluant hydrophile en phase liquide ne va plus provoquer une diminution de l'humidité relative $\varphi1$ de l'air humide introduit dans l'espace de tête. Aussi, dans ce cas de figure, le deuxième échantillon va progressivement présenter une humidité relative $\varphi2$ sensiblement égale à $\varphi1$, de sorte que le bruit de mesure associé à la différence $\Delta\varphi$ non nulle peut présenter une intensité diminuant dans le temps. Quoi qu'il en soit, les différentes caractérisations successives se traduiront par des signatures qui ne seront alors pas identiques au cours du temps (dérive temporelle des signatures).

Cette problématique de bruit de mesure lié à $\Delta\varphi$ est particulièrement importante lorsque le procédé de caractérisation est effectué à partir de signaux utiles $Su_k(t)$, c'est-à-dire qu'il comporte une étape de soustraction de la valeur de référence $\tilde{S}_k^{ref}$ (*baseline*) au signal de mesure $S_k(t)$ correspondant. En effet, le but de cette étape consiste à écarter de la caractérisation des composés d'intérêt l'effet associé à leur environnement et notamment l'effet du gaz porteur. Or, il apparaît que cette valeur de référence $\tilde{S}_k^{ref}$ est représentative du gaz porteur lors de la phase initiale P1, mais n'est plus nécessairement représentative du gaz porteur lors de la phase de caractérisation P2 puisque les propriétés physiques de ce gaz porteur dans la chambre de mesure ont pu changer (variation de l'humidité relative).

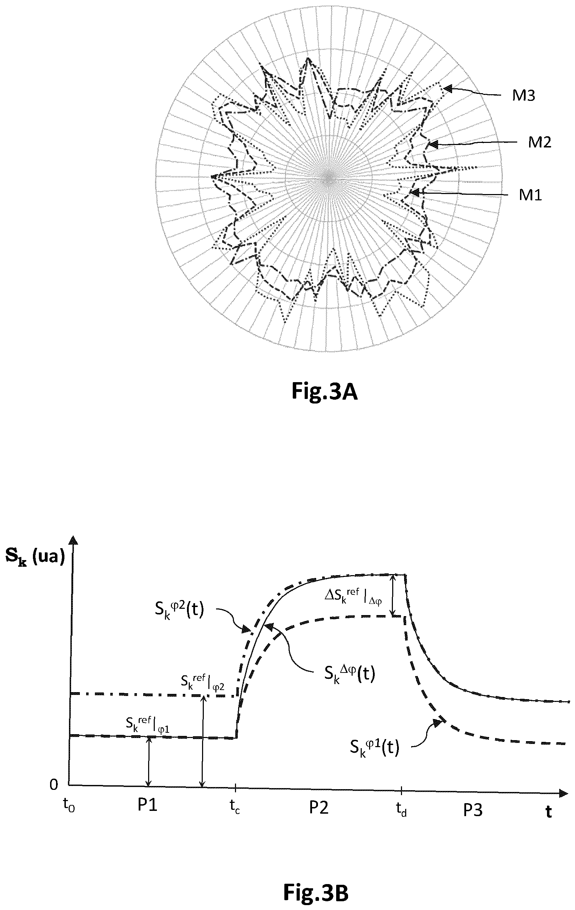

La figure 3A illustre trois motifs d'interaction, ou signatures, traduisant la caractérisation de différents échantillons gazeux, cette caractérisation étant effectuée par un procédé de caractérisation selon un exemple de l'art antérieur. Ces motifs d'interaction M1, M2 et M3 sont ici des représentations sous la forme d'un diagramme en radar des valeurs d'équilibres (stationnaires) déterminées à partir des sensorgrammes $Su_k(t)$ dans le régime stationnaire d'équilibre P2.2. Ils permettent de mettre en évidence l'effet de la différence d'humidité relative $\Delta\varphi$ sur la caractérisation des composés d'intérêt. Pour obtenir ces motifs d'interaction M1, M2, M3, le gaz porteur est identique pour les trois essais et correspond à de l'air humide présentant une humidité relative initiale $\varphi1$ de 12% environ.

Une première signature M1 correspond à un échantillon gazeux formé d'air humide avec une humidité relative $\varphi2$ égale à 50% environ et dont les composés d'intérêt sont des molécules de butanol. La mise en oeuvre du procédé de caractérisation présente ainsi une variation relativement importante de l'humidité relative dans la chambre de mesure, qui passe ici de $\varphi1$ égale à 12% environ lors de la phase initiale P1, à $\varphi2$ égale à

50% environ lors de la phase de caractérisation. Aussi, la valeur de référence $\tilde{S}_k^{ref}$ est déterminée pour un premier échantillon gazeux (air humide à $\varphi1$ de 12%) et la valeur d'équilibre est déterminée pour le deuxième échantillon gazeux (air humide à $\varphi2$ de 50% avec les composés d'intérêt) en soustrayant cette valeur de référence $S_k^{ref}$. Cette différence d'humidité relative $\Delta\varphi$ forme ainsi un bruit de mesure dont il importe de limiter l'effet pour que le motif d'interaction M1 soit effectivement représentatif uniquement des molécules de butanol.

Une deuxième signature M2 correspond à un deuxième échantillon gazeux formé d'air humide avec une humidité relative $\varphi2$ sensiblement égale à $\varphi1$ (soit 12%), et dont les composés d'intérêt sont également des molécules de butanol. La mise en oeuvre du procédé de caractérisation permet, en soustrayant la valeur de référence $\tilde{S}_k^{ref}$ associée au premier échantillon gazeux (air humide à $\varphi1$), et dans la mesure où la variation d'humidité relative $\Delta\varphi$ est nulle, d'écarter l'effet de l'environnement gazeux pour ainsi caractériser les interactions seules des composés d'intérêt avec les récepteurs. Aussi, la signature M2 est représentative des seuls composés d'intérêt puisqu'il n'y a pas de bruit de mesure associé à une variation d'humidité relative $\Delta\varphi$. On remarque que la signature M1 ne se superpose pas à la signature M2, traduisant bien la présence du bruit de mesure associé à $\Delta\varphi$ dans le cas de M1. Il importe donc d'être en mesure de corriger la signature M1 pour tendre vers la signature M2, laquelle est seule représentative des composés d'intérêt, quand bien même il y a une différence d'humidité relative $\Delta\varphi$ dans la chambre de mesure entre la phase initiale P1 et la phase de caractérisation P2.

La troisième signature M3 correspond à un échantillon gazeux formé seulement d'air humide avec une humidité relative $\varphi2$ égale à 50% environ. Ici, on mesure l'impact seul de la variation de l'humidité relative $\Delta\varphi$ sur la caractérisation de l'air humide par le nez électronique, en l'absence de composés d'intérêt. Il apparaît que l'augmentation d'humidité relative $\Delta\varphi$ entre la phase initiale P1 et la phase de caractérisation P2 se traduit par une augmentation de la variation de réflectivité $\Delta\%R_k$ des sites sensibles $6_k$. On remarque que la signature M1 (air humide avec $\Delta\varphi$ non nulle et composé d'intérêt) est située entre la signature M2 (air humide avec $\Delta\varphi$ nulle et composés d'intérêt) et la signature M3 (air humide avec $\Delta\varphi$ non nulle sans composés d'intérêt), montrant bien l'effet du bruit de mesure associé à la différence d'humidité relative $\Delta\varphi$ non nulle sur la signature des composés d'intérêt. Il importe donc d'être en mesure de limiter voire écarter ce bruit de mesure pour améliorer la qualité de la caractérisation des composés d'intérêt.

La figure 3B illustre des exemples de signaux de mesure $S_k(t)$ pour différents échantillons gazeux, mettant ainsi également en évidence l'impact du bruit de mesure associé à la variation d'humidité relative $\Delta\varphi$ sur la caractérisation des composés d'intérêt. Dans ces exemples, le gaz porteur est de l'air humide.

Le signal de mesure $S_k^{\varphi1}(t)$ correspond au cas où l'humidité relative dans la chambre de mesure reste constante et égale à $\varphi 1$ lors de la phase initiale P1 et de la phase de caractérisation P2. La variation d'humidité relative $\Delta\varphi$ est alors nulle. Il présente une valeur de référence $S_k^{ref}|_{\varphi 1}$ non nulle, qui correspond à la réponse du nez électronique lorsque le premier échantillon gazeux (air humide à $\varphi 1$ sans composés d'intérêt) est dans la chambre de mesure.

Le signal de mesure $Sk^{\varphi 2}(t)$ correspond au cas où l'humidité relative dans la chambre de mesure reste constante et égale à $\varphi 2$ lors de la phase initiale P1 et de la phase de caractérisation P2. La variation d'humidité relative $\Delta\varphi$ est alors nulle. L'humidité relative $\varphi 2$ est ici supérieure à $\varphi 1$. Il présente une valeur de référence $S_k^{ref}|_{\varphi 2}$ non nulle et différente de $S_k^{ref}|_{\varphi 1}$, qui correspond à la réponse du nez électronique lorsque le premier échantillon gazeux (air humide à $\varphi 2$ sans composés d'intérêt) est dans la chambre de mesure.

Le signal de mesure $S_k^{\Delta\varphi}(t)$ correspond au cas où l'humidité relative dans la chambre de mesure n'est pas constante, et passe de la valeur $\varphi 1$ lors de la phase initiale P1 à la valeur $\varphi 2$ de la phase de caractérisation P2. La variation d'humidité relative $\Delta\varphi$ est alors non nulle, et ici positive. Il présente la même valeur de référence $S_k^{ref}|_{\varphi 1}$ que pour le signal de mesure $S_k^{\varphi 1}(t)$ dans la mesure où l'échantillon gazeux présentait l'humidité relative $\varphi 1$ lors de la phase initiale P1. En revanche, il présente la même valeur d'équilibre que le signal de mesure $S_k^{\varphi 2}(t)$ dans la mesure où l'échantillon gazeux présentait l'humidité relative $\varphi 2$ lors de la phase de caractérisation P2. Aussi, le signal de mesure $S_k^{\Delta\varphi}(t)$ passe progressivement du signal de mesure $S_k^{\varphi 1}(t)$ lors de la phase initiale P1 au signal de mesure $S_k^{\varphi 2}(t)$ lors de la phase de caractérisation P2. Le bruit de mesure présente alors une intensité de l'ordre de $\Delta S_k^{ref}|_{\Delta\varphi}$ correspondant à la différence entre $S_k^{ref}|_{\varphi 2}$ et $S_k^{ref}|_{\varphi 1}$. Il s'agit alors, pour caractériser les composés d'intérêt seuls, de corriger le signal de mesure $S_k^{\Delta\varphi}(t)$ du bruit de mesure $\Delta S_k^{ref}|_{\Delta\varphi}$ et de lui soustraire la valeur de référence $S_k^{ref}|_{\varphi 1}$.

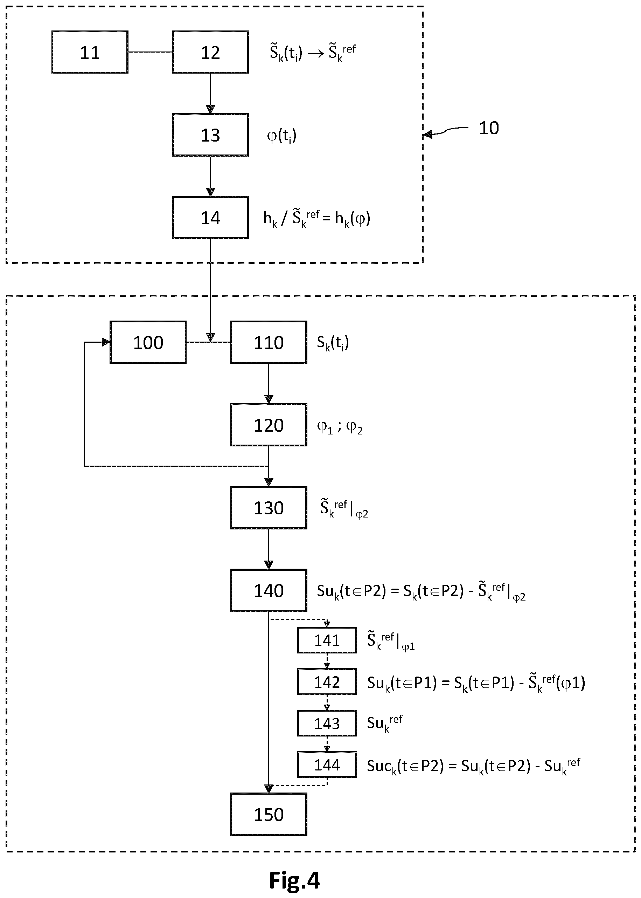

La figure 4 illustre un organigramme d'un procédé de caractérisation de composés d'intérêt selon un premier mode de réalisation, dans lequel le bruit de mesure lié à une différence d'humidité relative $\Delta\varphi$ non nulle est réduit voire écarté, la différence d'humidité relative $\Delta\varphi$ dans la chambre de mesure étant définie entre une valeur $\varphi 1$ lors de la phase initiale P1 et une valeur $\varphi 2$ différente de $\varphi 1$ lors de la phase de caractérisation P2. Dans ce mode de réalisation, le signal utile est corrigé à partir d'une estimation d'une valeur de référence $\tilde{S}_k^{ref}|_{\varphi 2}$ associée au premier échantillon gazeux pour l'humidité relative $\varphi 2$, cette estimation étant obtenue à partir d'une fonction de calibration $h_k$.

Dans cet exemple, la chambre de mesure comporte une pluralité de sites sensibles $6_k$ distincts, mais en variante elle peut n'en comporter qu'un seul. Par ailleurs, le signal de mesure détecté par le capteur d'image est une partie du signal optique d'excitation réfléchie par les sites sensibles $6_k$, mais en variante il peut s'agir d'une partie transmise. Le nez électronique selon un mode de réalisation comporte un capteur d'humidité 9 adapté à mesurer l'humidité relative dans la chambre de mesure. Ce capteur d'humidité 9 peut être disposé dans la chambre de mesure ou dans les conduits fluidiques amont ou aval. Il est connecté à l'unité de traitement qui détermine la différence d'humidité relative $\Delta\varphi$ entre la phase initiale P1 et la phase de caractérisation P2.

Lors d'une phase préalable 10 de calibration, on détermine une fonction de calibration $h_k$ associée à chaque site sensible $6_k$. Cette fonction de calibration exprime une variation d'un paramètre représentatif du signal de mesure associé au premier échantillon gazeux formé du seul gaz porteur (sans les composés d'intérêt) en fonction de l'humidité relative $\varphi$. Plus précisément, le paramètre représentatif est ici une valeur de référence du signal de mesure lorsque le premier échantillon gazeux est présent dans la chambre de mesure. On note ici $\tilde{S}_k^{ref}$ la valeur de référence estimée du signal de mesure $S_k(t)$ déterminée lors de la phase de calibration. Plus précisément, on place le signe tilde sur la lettre S lorsque le signal de mesure et sa valeur de référence sont associés à la fonction de calibration. La fonction de calibration est une fonction continue qui peut être polynomiale, logarithmique ou autre. Elle est paramétrisée lors de la phase 10 de calibration, comme détaillé plus loin.

Lors d'une première étape 100, on effectue l'étape d'injection fluidique dans la chambre de mesure du nez électronique. Cette étape comporte une première phase initiale P1 d'injection du premier échantillon gazeux (gaz porteur sans les composés d'intérêt), une deuxième phase P2 dite de caractérisation au cours de laquelle est injecté le deuxième échantillon gazeux (gaz porteur avec les composés d'intérêt), puis une troisième phase de dissociation P3. Les deux échantillons gazeux présentent des humidités relatives différentes, notées $\varphi 1$ pour le premier échantillon gazeux et $\varphi 2$ pour le deuxième.

Lors d'une étape 110, on détermine, pour chaque site sensible $6_k$ allant de 1 à N, à l'instant courant $t_i$, un signal de mesure $S_k(t_i)$ représentatif de la réflectivité $\%R_k(t_i)$ du site sensible $6_k$ considéré, et donc représentatif en outre de la réponse du nez électronique en présence de l'un puis de l'autre des échantillons gazeux dans la chambre de mesure.

Pour cela, on acquiert, au cours de l'étape d'injection 100, une pluralité d'images dites élémentaires $Ie_m$ des N sites sensibles $6_k$. Plus précisément, on éclaire les sites sensibles $6_k$ par un signal optique d'excitation apte à y générer des plasmons de surface, et on détecte la partie réfléchie du signal optique d'excitation. Le capteur d'image 8 est connecté à l'unité de traitement, qui stocke les images acquises.

Le capteur d'image 8 acquiert, sur une durée $\Delta t$ séparant deux instants de mesure successifs $t_{i-1}$ et $t_i$, une pluralité d'images dites élémentaires $Ie_m$ de la matrice des N sites sensibles distincts, m étant le rang d'acquisition de l'image élémentaire $Ie$, à une fréquence d'échantillonnage $f_e$. La fréquence d'échantillonnage $f_e$ peut être de 10 images par seconde, et la durée $\Delta t$ d'acquisition peut être de quelques secondes, par exemple 4s.

Pour chaque image élémentaire $Ie_m$, l'unité de traitement détermine une valeur d'intensité optique élémentaire $(I_k)_m$ en faisant la moyenne de l'intensité optique $(I_k(i,j))_m$ acquise par chaque pixel i,j associé à un même site sensible $6_k$, et en calcule une valeur moyenne $(\overline{I_k})_{\Delta t}$ sur la durée $\Delta t$ d'acquisition. Cette valeur moyenne $(\overline{I_k})_{\Delta t}$ correspond alors au signal de mesure $S_k(t_i)$, à l'instant courant $t_i$, associé au site sensible $6_k$.

Cette étape 110 d'acquisition et de détermination des signaux de mesure $S_k(t_i)$ est effectuée au cours de l'étape d'injection fluidique 100, et réitérée pour plusieurs instants de mesure $t_i$ successifs. A chaque itération i est associé un instant de mesure $t_i$ également appelé instant courant.

Lors d'une étape 120, on mesure l'humidité relative dans la chambre de mesure lors des phases P1 et P2. Pour cela, le capteur d'humidité 9 mesure l'humidité relative $\varphi$ lors des phases P1 et P2 et transmet les valeurs mesurées à l'unité de traitement. L'humidité relative $\varphi 2$ est ici différente de la valeur $\varphi 1$. Les valeurs d'humidité relative $\varphi 1$ et $\varphi 2$ peuvent être une valeur moyenne de l'humidité relative au cours de cette phase considérée ou au cours d'une durée déterminée. L'humidité relative $\varphi 1$ est de préférence une valeur moyenne calculée sur une durée qui précède directement l'instant $t_c$ et donc la phase de caractérisation P2. L'humidité relative $\varphi 2$ est de préférence une valeur moyenne calculée sur une durée située lors du régime stationnaire P2.2, par exemple pendant une durée qui précède directement l'instant $t_d$ et donc la phase de dissociation P3.

Lors d'une étape 130, on détermine une valeur de référence $\tilde{S}_k^{ref}|_{\varphi 2}$ représentative du signal de mesure associé au premier échantillon gazeux pour une humidité relative $\varphi 2$. Cette valeur de référence $\tilde{S}_k^{ref}|_{\varphi 2}$ est calculée à partir de la fonction de calibration $h_k$ et à partir de la valeur mesurée $\varphi 2$. Autrement dit :

$$\tilde{S}_k^{ref}|_{\varphi 2} = h_k(\varphi 2).$$

Lors d'une étape 140, on corrige le signal de mesure $S_k(t_i)$ associé au deuxième échantillon gazeux, c'est-à-dire pour $t_i$ appartenant à la phase P2, par soustraction de la valeur de référence $\tilde{S}_k^{ref}|_{\varphi 2}$ déterminée. On obtient ainsi un signal utile $Su_k(t_i \in P2)$. Ainsi, dans le cadre de ce mode de réalisation, on calcule le signal utile associé au deuxième échantillon gazeux, i.e. celui comprenant les composés d'intérêt mais ayant subi une variation d'humidité relative, par correction de son signal de mesure $S_k(t_i \in P2)$ lors de la phase P2 par la valeur de référence $\tilde{S}_k^{ref}|_{\varphi 2}$ issue de la fonction de calibration $h_k$ et non pas en lui soustrayant sa valeur de référence $S_k^{ref}$. En effet, cette valeur $S_k^{ref}$ est associée à l'humidité relative $\varphi 1$ du premier échantillon gazeux pendant la phase P1 alors que le signal de mesure du deuxième échantillon gazeux a subi le bruit de mesure lié à la différence $\Delta\varphi$. Aussi, soustraire au signal de mesure $S_k(t_i \in P2)$ la valeur de référence $S_k^{ref}$ ne permet pas de tenir compte du bruit de mesure. Alors que lui soustraire la valeur de référence $\tilde{S}_k^{ref}|_{\varphi 2}$ issue de la fonction de calibration $h_k$ permet de tenir compte de la différence de l'humidité relative $\Delta\varphi$.

Lors d'une étape 150, on caractérise les composés d'intérêt à partir des signaux utiles corrigés $Su_k(t_i \in P2)$. On extrait de ces signaux une valeur d'équilibre, i.e. stationnaire, pour fournir une représentation sous forme d'un histogramme, d'un diagramme en radar, ou autre, formant la signature des composés cibles.

Aussi, le procédé de caractérisation selon ce mode de réalisation permet d'améliorer la qualité de la caractérisation des composés d'intérêt, en limitant voire écartant le bruit de mesure lié à une variation de l'humidité relative entre les phases P1 et P2 est présent. Le signal utile $Su_k(t_i \in P2)$ permettant de caractériser les composés d'intérêt est calculé en corrigeant le signal de mesure $S_k(t_i \in P2)$ associé au deuxième échantillon gazeux par une valeur de référence $\tilde{S}_k^{ref}|_{\varphi 2}$ associée au premier échantillon gazeux et à l'humidité relative $\varphi 2$.

Cela revient à estimer la valeur de référence $\tilde{S}_k^{ref}|_{\varphi 2}$ qu'aurait le premier échantillon gazeux pour l'humidité relative $\varphi 2$, puis à soustraire cette valeur au signal de mesure $S_k(t_i)$ détecté à l'étape 110. La caractérisation des composés d'intérêt est alors rendue plus juste et précise, dans la mesure où elle porte sur les composés d'intérêt seul et non pas ou peu sur le gaz porteur qui a connu une variation de son humidité relative.

Les étapes 141 à 144 peuvent être effectuées de manière avantageuse. Elles permettent d'améliorer encore la qualité de la caractérisation des composés d'intérêt, dans le cas où le nez électronique présente une dérive de capteur, c'est-à-dire une variation du signal de mesure émis par le nez électronique alors que les composés d'intérêt et les conditions opératoires sont les mêmes. Cette dérive de capteur a lieu ici entre la phase de calibration 10 et la phase de caractérisation 100-150.

Lors de l'étape 141, on détermine la valeur de

référence $\tilde{S}_k^{ref}|_{\varphi 1}$ représentative du signal de mesure associé au premier échantillon gazeux pour une humidité relative $\varphi 1$. Cette valeur de référence $\tilde{S}_k^{ref}|_{\varphi 1}$ est calculée à partir de la fonction de calibration $h_k$ et à partir de la valeur mesurée $\varphi 1$. Autrement dit, $\tilde{S}_k^{ref}|_{\varphi 1} = h_k(\varphi 1)$.

Lors de l'étape 142, on corrige le signal de mesure $S_k(t_i)$ associé au premier échantillon gazeux, c'est-à-dire pour $t_i$ appartenant à la phase P1, par soustraction de la valeur de référence $\tilde{S}_k^{ref}|_{\varphi}$ déterminée. On obtient ainsi un signal utile $Su_k(t_i \in P1)$, tel que $Su_k(t_i \in P1) = S_k(t_i) - \tilde{S}_k^{ref}|_{\varphi}$. Du fait de la dérive de capteur, le signal utile $Su_k(t_i \in P1)$ n'est pas sensiblement nul alors qu'il le devrait.

Lors de l'étape 143, on détermine la valeur de référence $Su_k^{ref}$ du signal utile $Su_k(t_i \in P1)$. Il s'agit par exemple de la valeur moyenne de ce signal utile sur une durée prédéfinie, avant l'instant $t_c$ et donc avant la phase P2.

Lors de l'étape 144, on corrige le signal utile $Su_k(t_i \in P2)$ associé au deuxième échantillon gazeux en lui soustrayant la valeur de référence $Su_k^{ref}$ déterminée. On obtient ainsi un signal utile corrigé $Suc_k(t_i \in P2)$ qui fait abstraction de la dérive du capteur.

Lors de l'étape 150, on caractérise les composés d'intérêt à partir du signal utile corrigé $Suc_k(t_i \in P2)$. Dans la mesure où la dérive du capteur est corrigée, on obtient une caractérisation des composés d'intérêt d'une qualité améliorée.

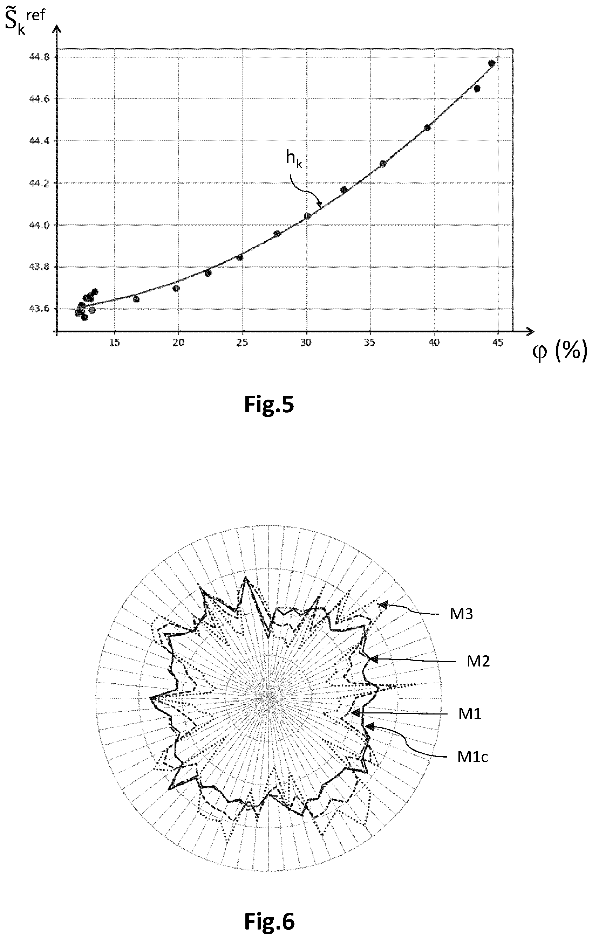

La phase de calibration 10 est maintenant décrite, en référence à la figure 4 et à la figure 5, laquelle illustre un exemple de variation de la valeur de référence $\tilde{S}_k^{ref}$ associé à un premier échantillon gazeux en fonction de l'humidité relative $\varphi$. Lors de cette phase, on utilise le signe tilde sur la lettre S pour différentier les signaux de mesure acquis lors de cette phase de ceux acquis lors de la phase de caractérisation.

Lors d'une étape 11, on injecte le premier échantillon gazeux dans la chambre de mesure. Le premier échantillon gazeux est donc formé du gaz porteur mais ne contient pas les composés d'intérêt. Il présente une humidité relative $\varphi$ non nulle, qui varie dans le temps, de préférence par paliers.

Lors d'une étape 12, on détermine, pour chaque site sensible $6_k$ allant de 1 à N, à l'instant courant $t_i$, un signal de mesure $\tilde{S}_k(t_i)$ représentatif ici de la réflectivité $\%R_k(t_i)$ du site sensible $6_k$ considéré, et donc représentatif en outre de la réponse du nez électronique en présence du premier échantillon gazeux dans la chambre de mesure. Cette étape est similaire à l'étape 120 et n'est donc pas décrite à nouveau. Dans la mesure où le premier échantillon gazeux ne comporte pas de composés d'intérêt, le signal de mesure $\tilde{S}_k(t_i)$ ne présente pas le régime transitoire d'assimilation P2.1 et le régime stationnaire d'équilibre P2.2. On peut donc déterminer une valeur de référence $\tilde{S}_k^{ref}|_{\varphi}$ associée à une valeur d'humidité relative $\varphi$ donnée. Il s'agit de préférence d'une valeur moyenne de $\tilde{S}_k(t_i)$ sur une durée prédéfinie, où $\varphi$ est de préférence constante.

Lors de l'étape 13, on mesure l'humidité relative $\varphi(t_i)$ au cours du temps à l'aide du capteur d'humidité 9.

Lors de l'étape 14, on détermine la fonction de calibration $h_k$ à partir des valeurs de référence $\tilde{S}_k^{ref}|_{\varphi}$ déterminées et des valeurs d'humidité relative $\varphi$ mesurées. La fig.5 illustre un exemple de fonction de calibration $h_k$ qui illustre la variation de la valeur de référence $\tilde{S}_k^{ref}$ associée au premier échantillon gazeux (air humide, par exemple) en fonction de l'humidité relative. Dans cet exemple, la fonction de calibration est une fonction polynomiale dont la paramétrisation, c'est-à-dire la détermination de l'ordre n du polynôme et des coefficients, est effectuée ici par régression polynomiale. D'autres types de fonctions de calibration peuvent être utilisés, comme les fonctions logarithmiques, les réseaux de neurones à sigmoïdes, les mélanges gaussiens, etc. Ainsi, à la suite de la phase de calibration, on obtient une fonction de calibration $h_k$ associée à chaque site sensible $6_k$ qui permet de déterminer la valeur de référence $\tilde{S}_k^{ref}|_{\varphi}$ associée au premier échantillon gazeux (air humide, par ex.) pour une humidité relative $\varphi$ donnée.

La figure 6 illustre les trois signatures M1, M2 et M3 représentées sur la fig.3A. La signature M1c correspond au même deuxième échantillon gazeux que pour la signature M1, c'est-à-dire un échantillon gazeux formé d'air humide avec une humidité relative $\varphi 2$ égale à 50% environ (donc avec une variation d'humidité relative $\Delta\varphi$) et dont les composés d'intérêt sont des molécules de butanol. Alors que la signature M1 est obtenue par un procédé de caractérisation selon un exemple de l'art antérieur, la signature M1c est obtenue par le procédé de caractérisation illustré sur la fig.4. On remarque que la signature M1c est superposée à la signature M2 qui correspond à une absence de variation d'humidité relative $\Delta\varphi$ entre les phases P1 et P2. Aussi, le procédé de caractérisation selon ce mode de réalisation de l'invention permet effectivement de réduire voire d'écarter le bruit de mesure associé à une variation non nulle d'humidité relative entre les phases P1 et P2.

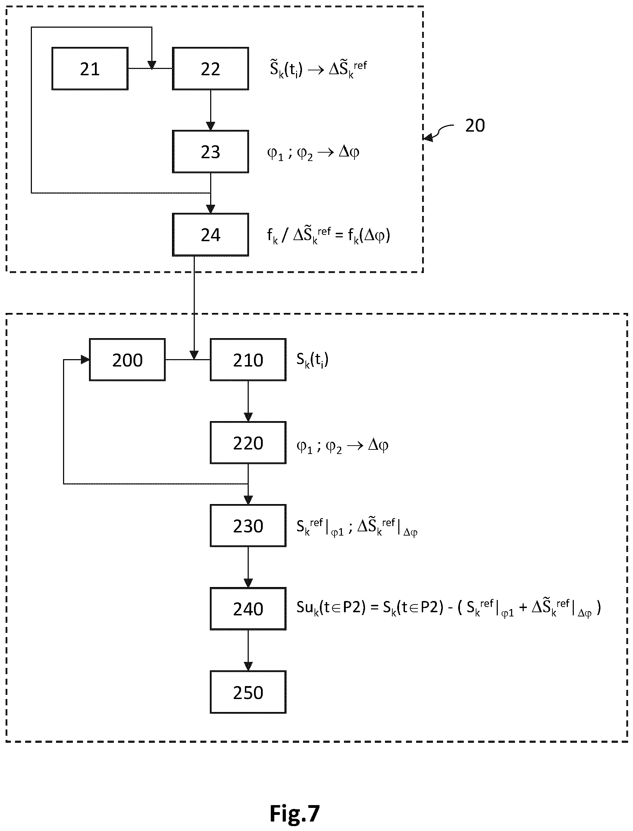

La figure 7 illustre un organigramme d'un procédé de caractérisation de composés d'intérêt selon un deuxième mode de réalisation, dans lequel le bruit de mesure lié à une différence d'humidité relative $\Delta\varphi$ non

nulle entre les phases P1 et P2 est réduit voire écarté, la différence d'humidité relative $\Delta\varphi$ dans la chambre de mesure étant définie entre une valeur $\varphi1$ lors de la phase initiale P1 et une valeur $\varphi2$ différente de $\varphi1$ lors de la phase de caractérisation P2. Ce procédé se distingue de celui décrit sur la fig.4 essentiellement en ce que le signal utile est corrigé en utilisant notamment une estimation de la différence de valeur de référence $\Delta\tilde{S}_k^{\mathrm{ref}}|_{\Delta\varphi}$ obtenue à partir d'une fonction de calibration $f_k$.

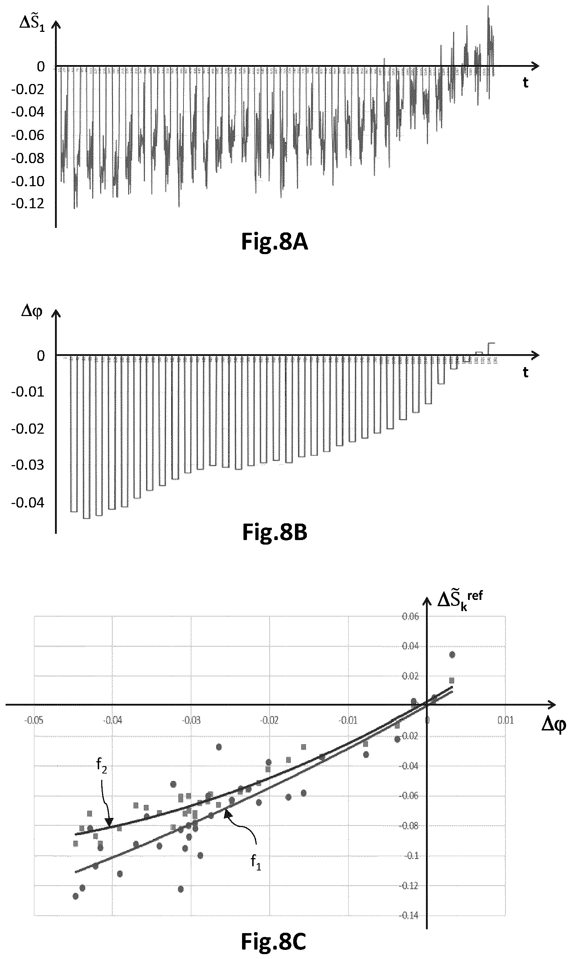

Lors d'une phase de calibration 100, on détermine la fonction de calibration $f_k$. Cette phase est illustrée sur les figures 8A à 8C. L'objectif est de déterminer une fonction de calibration $f_k$ exprimant la variation de la différence de valeur de référence $\Delta\tilde{S}_k^{\mathrm{ref}}|_{\Delta\varphi}$ en fonction d'une différence d'humidité relative $\Delta\varphi$.

Lors d'une étape 21, on effectue plusieurs cycles d'injection du premier échantillon gazeux dans la chambre de mesure. Le premier échantillon gazeux est donc formé du gaz porteur mais ne contient pas les composés d'intérêt. Chaque cycle est formé d'une première injection du premier échantillon gazeux à une humidité relative $\varphi1$ constante et non nulle, suivie d'une deuxième injection du premier échantillon gazeux à une humidité relative $\varphi2$ constante et différente de $\varphi1$. Au cours des différents cycles, l'humidité relative $\varphi2$ varie, de sorte que l'on obtient plusieurs valeurs de la différence d'humidité relative $\Delta\varphi = \varphi2 - \varphi1$.

Lors d'une étape 22, on acquiert le signal de mesure $\tilde{S}_k(t_i)$ lors de l'étape 21. La fig.8A illustre plus précisément l'écart $\Delta\tilde{S}_1(t_i)$ entre les signaux de mesure associés aux deux injections de chaque cycle pour un site sensible $6_1$ en fonction du temps. Plus précisément, au signal de mesure de la deuxième injection, on soustrait le signal de mesure de la première injection. Aussi, comme le montre la fig.8A, l'écart $\Delta\tilde{S}_1(t_i)$ présente une valeur nulle lors des premières injections et une valeur non nulle lors des deuxièmes injections.

On détermine alors la différence des valeurs de référence $\Delta\tilde{S}_k^{\mathrm{ref}}$ entre les première et deuxième injections de chaque cycle. Il s'agit de déterminer la valeur de référence $\tilde{S}_k^{\mathrm{ref}}$ pour les première et deuxième injections de chaque cycle et d'en faire la différence. La valeur de référence $\tilde{S}_k^{\mathrm{ref}}$ est de préférence une valeur moyenne du signal de mesure $\tilde{S}_k(t_i)$ sur une durée prédéfinie.

Lors d'une étape 23, on mesure la différence d'humidité relative $\Delta\varphi$ entre les première et deuxième injections de chaque cycle. La fig.8B illustre la variation de la différence d'humidité relative $\Delta\varphi$ en fonction du temps. On remarque que la différence $\Delta\varphi$ est nulle pour chaque première injection, et qu'elle est non nulle et varie dans le temps d'une deuxième injection à l'autre.

Lors d'une étape 24, on détermine la fonction de calibration $f_k$ à partir des valeurs déterminées de la différence de valeur de référence $\Delta\tilde{S}_k^{\mathrm{ref}}$ et des valeurs mesurées de la différence d'humidité relative $\Delta\varphi$. La fig.8C illustre un exemple de deux fonctions de calibration $f_1$, $f_2$ qui illustre la variation de la différence de valeur de référence $\Delta\tilde{S}_k^{\mathrm{ref}}$ associé au premier échantillon gazeux (air humide, par exemple) en fonction de la différence d'humidité relative $\Delta\varphi$. Dans cet exemple, la fonction de calibration est une fonction polynomiale dont la paramétrisation, c'est-à-dire la détermination de l'ordre n du polynôme et des coefficients, est effectuée ici par régression polynomiale. D'autres types de fonctions de calibration peuvent être utilisés, comme les fonctions logarithmiques, les réseaux de neurones à sigmoïdes, les mélanges gaussiens, etc., comme pour le mode de réalisation de la fig.4. Ainsi, à la suite de la phase de calibration, on obtient une fonction de calibration $f_k$ associée à chaque site sensible $6_k$ qui permet de déterminer la différence de valeur de référence $\Delta\tilde{S}_k^{\mathrm{ref}}$ associée au premier échantillon gazeux (air humide, par ex.) pour une différence d'humidité relative $\Delta\varphi$ donnée.

Puis, la phase de calibration 200 à 250 est effectuée. Lors d'une étape 200, on effectue l'étape d'injection fluidique dans la chambre de mesure du nez électronique. Cette étape comporte une première phase initiale P1 d'injection du premier échantillon gazeux (gaz porteur sans les composés d'intérêt), une deuxième phase P2 dite de caractérisation au cours de laquelle est injecté le deuxième échantillon gazeux (gaz porteur avec les composés d'intérêt), puis une troisième phase de dissociation P3. Les deux échantillons gazeux présentent des humidités relatives différentes, notées $\varphi1$ pour le premier échantillon gazeux et $\varphi2$ pour le deuxième.

Lors d'une étape 210, on détermine, pour chaque site sensible $6_k$ allant de 1 à N, à l'instant courant $t_i$, un signal de mesure $S_k(t_i)$ représentatif de la réponse du nez électronique en présence de l'un puis de l'autre des échantillons gazeux dans la chambre de mesure. Cette étape est similaire à l'étape 110 et n'est pas décrite en détail à nouveau.

Lors d'une étape 220, on mesure l'humidité relative dans la chambre de mesure lors des phases P1 et P2. Pour cela, le capteur d'humidité 9 mesure l'humidité relative $\varphi$ lors des phases P1 et P2 et transmet les valeurs mesurées à l'unité de traitement. L'humidité relative $\varphi2$ est ici différence de la valeur $\varphi1$. L'unité de traitement détermine ensuite la différence d'humidité relative $\Delta\varphi = \varphi2 - \varphi1$.

Lors d'une étape 230, on détermine l'estimation de la différence de valeur de référence $\Delta\tilde{S}_k^{\mathrm{ref}}|_{\Delta\varphi}$ à partir de la différence d'humidité relative $\Delta\varphi$ mesurée. Cette

différence de valeur de référence $\Delta\tilde{S}_k^{ref}|_{\Delta\varphi}$, comme l'illustre la fig.3B, correspond à l'impact sur le signal de mesure associé au deuxième échantillon gazeux qu'induit la différence d'humidité relative $\Delta\varphi$ entre les phases P1 et P2. Cette différence de valeur de référence $\Delta\tilde{S}_k^{ref}|_{\Delta\varphi}$ est calculée à partir de la fonction de calibration $f_k$ et à partir de la différence $\Delta\varphi$ mesurée. Puis, on détermine également la valeur de référence $S_k^{ref}|_{\varphi1}$ associée au premier échantillon gazeux à partir du signal de mesure $S_k(t_i \in P1)$. Il s'agit de préférence d'une valeur moyenne du signal de mesure $S_k(t_i \in P1)$ pendant la phase P1 sur une durée prédéfinie.

Lors d'une étape 240, on corrige le signal de mesure $S_k(t_i)$ associé au deuxième échantillon gazeux, c'est-à-dire pour $t_i$ appartenant à la phase P2, par soustraction de l'estimation de la différence de valeur de référence $\Delta\tilde{S}_k^{ref}|_{\Delta\varphi}$ pour la différence d'humidité relative $\Delta\varphi$ mesurée, et par soustraction de la valeur de référence $S_k^{ref}|_{\varphi1}$ associée au premier échantillon gazeux. On obtient ainsi un signal utile $Su_k(t_i \in P2)$. Autrement dit,

$$Su_k(t_i \in P2) = S_k(t_i \in P2) - [\Delta\tilde{S}_k^{ref}|_{\Delta\varphi} + S_k^{ref}|_{\varphi1}]$$.

Ainsi, dans le cadre de ce mode de réalisation, on calcule le signal utile associé au deuxième échantillon gazeux, i.e. celui comprenant les composés d'intérêt mais ayant subi une variation d'humidité relative, par correction de son signal de mesure $S_k(t_i \in P2)$ en lui soustrayant d'une part l'impact $\Delta\tilde{S}_k^{ref}|_{\Delta\varphi}$ qu'induit la différence d'humidité relative $\Delta\varphi$, et d'autre part la valeur de référence $S_k^{ref}|_{\varphi1}$. Aussi, ce mode de réalisation permet d'écarter le bruit de mesure associé à la différence d'humidité relative $\Delta\varphi$, et également d'écarter l'éventuelle dérive de capteur.

Lors d'une étape 250, on caractérise les composés d'intérêt à partir des signaux utiles $Su_k(t_i \in P2)$. On extrait de ces signaux une valeur d'équilibre, i.e. stationnaire, pour fournir une représentation sous forme d'un histogramme, d'un diagramme en radar, ou autre, formant la signature des composés cibles.

Aussi, le procédé de caractérisation selon ce mode de réalisation permet également d'améliorer la qualité de la caractérisation des composés d'intérêt, en limitant voire écartant le bruit de mesure lié à une variation de l'humidité relative entre les phases P1 et P2 est présent, mais également en limitant voire écartant une éventuelle dérive de capteur entre la phase de calibration et la phase de caractérisation. La caractérisation des composés d'intérêt est alors rendue plus juste et précise, dans la mesure où elle porte sur les composés d'intérêt seul et non pas ou peu sur le gaz porteur qui a connu une variation de son humidité relative.

Des modes de réalisation particuliers viennent d'être décrits. Différentes variantes et modifications apparaîtront à l'homme du métier.

**Revendications**

1. Procédé de caractérisation de composés d'intérêt introduits dans une chambre de mesure (4) d'un nez électronique comportant au moins un site sensible ($6_k$) ayant des récepteurs avec lesquels les composés d'intérêt sont aptes à interagir par adsorption/désorption, le procédé comportant les étapes suivantes :

   ○ injection, dans la chambre de mesure (4) :

      • pendant une première phase P1, d'un premier échantillon gazeux formé d'un gaz porteur sans les composés d'intérêt, puis
      • pendant une deuxième phase P2, d'un deuxième échantillon gazeux formé d'au moins le gaz porteur et desdits composés d'intérêt ;

   ○ détermination, au cours desdites phases P1 et P2, d'un signal de mesure ($S_k(t_i)$) représentatif des interactions entre l'échantillon gazeux présent et les récepteurs du site sensible ($6_k$), à différents instants de mesure $t_i$, en réponse à un signal d'excitation émis au niveau du site sensible ($6_k$) ;
   ○ **caractérisé en ce qu'**il comporte en outre les étapes suivantes :

   ○ mesure (120 ; 220) de :

      • une valeur $\varphi1$ de l'humidité relative $\varphi$ dans la chambre de mesure lors de la première phase P1, et
      • une valeur $\varphi2$ de l'humidité relative $\varphi$ lors de la deuxième phase P2, $\varphi2$ étant différente de $\varphi1$ ;

   ○ détermination (130; 230) d'un paramètre correctif ($\tilde{S}_k^{ref}|_{\varphi2}$ ; $\Delta\tilde{S}_k^{ref}|_{\Delta\varphi}$) associé au site sensible ($6_k$), à partir :

      • d'au moins la valeur mesurée $\varphi2$ de l'humidité relative, et
      • d'une fonction de calibration ($f_k$, $h_k$) prédéterminée et associée au site sensible ($6_k$), la fonction de calibration ($f_k$, $h_k$) exprimant une variation d'un paramètre ($\tilde{S}_k^{ref}$ ; $\Delta\tilde{S}_k^{ref}$) représentatif du signal de mesure associé au premier échantillon gazeux en fonction de l'humidité relative ($\varphi$ ; $\Delta\varphi$) ;

○ détermination d'un signal utile ($Su_k(t_i\in P2)$) par correction du signal de mesure ($S_k(t_i\in P_2)$) associé au deuxième échantillon gazeux à partir d'au moins le paramètre correctif déterminé ( $\tilde{S}_k^{ref}|_{\varphi2}$ ; $\Delta\tilde{S}_k^{ref}|_{\Delta\varphi}$ ), et caractérisation des composés d'intérêt à partir du signal utile ($Su_k(t_i\in P2)$).

2. Procédé de caractérisation selon la revendication 1, comportant une phase (10) de détermination de la fonction de calibration notée $h_k$ comportant les étapes suivantes :

○ injection (11) du premier échantillon gazeux dans la chambre de mesure de sorte que l'humidité relative $\varphi$ varie progressivement, et mesure (13) de l'humidité relative $\varphi$ ;
○ détermination (12) d'un signal de mesure $\tilde{S}_k(t_i)$ au cours de l'étape d'injection (11), puis détermination d'une valeur de référence $\tilde{S}_k^{ref}$ à partir du signal de mesure $\tilde{S}_k(t_i)$ déterminé et pour chaque valeur d'humidité relative $\varphi$ mesurée ;
○ détermination (14) d'une fonction de calibration $h_k$ exprimant la variation de la valeur de référence $\tilde{S}_k^{ref}$ en fonction de l'humidité relative $\varphi$, à partir des valeurs de référence $\tilde{S}_k^{ref}$ déterminées et des valeurs d'humidité relative $\varphi$ mesurées.

3. Procédé de caractérisation selon la revendication 2, dans lequel le paramètre correctif est une valeur de référence $\tilde{S}_k^{ref}|_{\varphi2}$ représentative du signal de mesure $S_k(t_i)$ du premier échantillon gazeux pour l'humidité relative $\varphi2$ mesuré.

4. Procédé de caractérisation selon la revendication 2 ou 3, dans lequel :

○ l'étape de détermination (140) du signal utile comporte les sous-étapes suivantes :

• détermination (141) d'une valeur de référence $\tilde{S}_k^{ref}|_{\varphi1}$ représentative du signal de mesure du premier échantillon gazeux pour l'humidité relative $\varphi1$ mesuré, à partir de la fonction de calibration $h_k$ ;

• détermination (142) d'un signal utile $Su_k(t_i\in P1)$ associé au premier échantillon par soustraction au signal de mesure $S_k(t_i\in P1)$ déterminé lors de la phase initiale P1 de la valeur de référence $\tilde{S}_k^{ref}|_{\varphi1}$ déterminée ;
• détermination (143) d'une valeur de référence $Su_k^{ref}$ à partir du signal utile $Su_k(t_i\in P1)$ déterminé associé au premier échantillon ;
• détermination (144) d'un signal utile corrigé $Suc_k(t_i\in P2)$ associé au deuxième échantillon gazeux par soustraction au signal utile $Su_k(t_i\in P2)$ de la valeur de référence $Su_k^{ref}$ déterminée ;

○ puis, caractérisation (150) des composés d'intérêt à partir du signal utile corrigé $Suc_k(t_i\in P2)$ associé au deuxième échantillon gazeux.

5. Procédé de caractérisation selon la revendication 1, comportant une phase (20) de détermination de la fonction de calibration notée $f_k$ comportant les étapes suivantes :

○ injection (21) du premier échantillon gazeux dans la chambre de mesure de sorte que plusieurs cycles d'injection soient effectués, chaque cycle comportant une première injection du premier échantillon gazeux à une humidité relative $\varphi1$ suivie d'une deuxième injection du premier échantillon gazeux à une humidité relative $\varphi2$ différente de $\varphi1$, et détermination (23) de la différence d'humidité relative $\Delta\varphi$ entre $\varphi1$ et $\varphi2$ variant d'un cycle à l'autre ;
○ détermination (22) d'un signal de mesure $\tilde{S}_k(t_i)$ au cours des différents cycles d'injection, et détermination d'une différence de valeurs de référence $\Delta\tilde{S}_k^{ref}$ pour chaque cycle d'injection et pour chaque différence d'humidité relative $\Delta\varphi$ déterminée, à partir du signal de mesure $\tilde{S}_k(t_i)$ déterminé au cours des différents cycles d'injection ;
○ détermination (24) de la fonction de calibration $f_k$ exprimant la variation de la différence de valeur de référence $\Delta\tilde{S}_k^{ref}$ en fonction de la différence d'humidité relative $\Delta\varphi$, à partir des valeurs déterminées de la différence de valeur de référence $\Delta\tilde{S}_k^{ref}$ et des valeurs déterminées de la différence d'humidité relative $\Delta\varphi$.

6. Procédé de caractérisation selon la revendication 5, dans lequel le paramètre correctif est la somme de :

○ une valeur de la différence de valeurs de ré-

férence $\Delta\tilde{S}_k^{ref}$ pour la différence d'humidité relative $\Delta\varphi$ déterminée à partir de l'humidité relative mesurée lors de la première phase P1 et de la deuxième phase $P_2$, et

      ○ une valeur de référence $S_k^{ref}|_{\varphi 1}$ associée au premier échantillon gazeux et déterminée à partir du signal de mesure $S_k(t_i \in P1)$ déterminé lors de la première phase P1.

7. Procédé de caractérisation selon la revendication 6, dans lequel l'étape de détermination (240) du signal utile comporte la soustraction du paramètre correctif au signal de mesure $S_k(t_i \in P_2)$ associé au deuxième échantillon gazeux et déterminé lors de la deuxième phase $P_2$.

8. Procédé de caractérisation selon l'une quelconque des revendications 1 à 7, dans lequel la fonction de calibration $(f_k; h_k)$ est une fonction polynomiale, logarithmique, ou exponentielle.

9. Procédé de caractérisation selon l'une quelconque des revendications 1 à 8, dans lequel la fonction de calibration $(f_k; h_k)$ est une fonction polynomiale d'ordre 2.

10. Procédé de caractérisation selon l'une quelconque des revendications 1 à 9, dans lequel le nez électronique comporte un dispositif de mesure des interactions entre les composés d'intérêt et les récepteurs de type optique à résonance des plasmons de surface.

11. Procédé de caractérisation selon l'une quelconque des revendications 1 à 10, dans lequel le nez électronique comporte un dispositif de mesure des interactions entre les composés d'intérêt et les récepteurs de type résistif, piézoélectrique, mécanique, acoustique ou optique.

## Patentansprüche

1. Verfahren zum Charakterisieren von Verbindungen von Interesse, die in eine Messkammer (4) einer elektronischen Nase eingebracht werden, welche mindestens eine sensible Stelle ($6_k$) beinhaltet, die Rezeptoren aufweist, mit denen die Verbindungen von Interesse durch Adsorption/Desorption zu interagieren imstande sind, wobei das Verfahren die folgenden Schritte beinhaltet:

    ○ Injizieren in die Messkammer (4):

        • in einer ersten Phase P1 einer ersten gasförmigen Probe, die aus einem Trägergas ohne Verbindungen von Interesse gebildet wird, danach
        • in einer zweiten Phase P2 einer zweiten gasförmigen Probe, die aus mindestens dem Trägergas und den Verbindungen von Interesse gebildet wird;

    ○ Bestimmen im Laufe der Phasen P1 und P2 eines Messsignals ($S_k(t_i)$), welches repräsentativ für die Interaktionen zwischen der vorhandenen gasförmigen Probe und den Rezeptoren der sensiblen Stelle ($6_k$) zu unterschiedlichen Messzeitpunkten $t_i$ ist, als Reaktion auf ein Erregungssignal, welches im Bereich der sensiblen Stelle ($6_k$) ausgegeben wird;

    ○ **dadurch gekennzeichnet, dass** es weiter die folgenden Schritte beinhaltet:

      ◦ Messen (120; 220) von:

        • einem Wert $\varphi 1$ der relativen Feuchtigkeit $\varphi$ in der Messkammer in der ersten Phase P1, und
        • einem Wert $\varphi 2$ der relativen Feuchtigkeit $\varphi$ in der zweiten Phase P2, wobei sich $\varphi 2$ von $\varphi 1$ unterscheidet;

      ◦ Bestimmen (130; 230) eines Berichtigungsparameters ($\tilde{S}_k^{ref}|_{\varphi 2}$; $\Delta\tilde{S}_k^{ref}|_{\Delta\varphi}$), welcher der sensiblen Stelle ($6_k$) zugeordnet ist, aus:

        • mindestens einem Messwert $\varphi 2$ der relativen Feuchtigkeit, und
        • einer vorbestimmten und der sensiblen Stelle ($6_k$) zugeordneten Kalibrierfunktion ($f_k$, $h_k$), wobei die Kalibrierfunktion ($f_k$, $h_k$) eine Variation eines Parameters ($\widetilde{S_k^{ref}}$; $\widetilde{\Delta S_k^{ref}}$) ausdrückt, der repräsentativ für das Messsignal ist, das der ersten gasförmigen Probe in Abhängigkeit von der relativen Feuchtigkeit ($\varphi$; $\Delta\varphi$) zugeordnet ist;

    ○ Bestimmen eines nützlichen Signals ($Su_k(t_i \in P_2)$) durch Berichtigen des Messsignals ($S_k(t_i \in P_2)$), welches der zweiten gasförmigen Probe zugeordnet ist, aus mindestens dem bestimmten Berichtigungsparameter ($\tilde{S}_k^{ref}|_{\varphi 2}$; $\Delta\tilde{S}_k^{ref}|_{\Delta\varphi}$), und Charakterisieren der Verbindungen von Interesse aus dem nützlichen Signal ($Su_k(t_i \in P_2)$).

**2.** Verfahren zum Charakterisieren nach Anspruch 1, welches eine Phase (10) zum Bestimmen der notierten Kalibrierfunktion $h_k$ beinhaltet, die folgenden Schritte beinhaltend:

o Injizieren (11) der ersten gasförmigen Probe in die Messkammer, sodass die relative Feuchtigkeit $\varphi$ nach und nach variiert, und Messen (13) der relativen Feuchtigkeit $\varphi$;

o Bestimmen (12) eines Messsignals $\widetilde{S_k}(t_i)$ im Laufe des Injektionsschrittes (11), danach Bestimmen eines Bezugswertes $\tilde{S}k^{ref}$ aus dem bestimmten Messsignal $\widetilde{S_k}(t_i)$ und für jeden gemessenen relativen Feuchtigkeitswert $\varphi$;

o Bestimmen (14) einer Kalibrierfunktion $h_k$, die die Variation des Bezugswertes $\tilde{S}k^{ref}$ in Abhängigkeit von der relativen Feuchtigkeit $\varphi$ ausdrückt, aus den bestimmten Bezugswerten $\tilde{S}k^{ref}$ und den gemessenen relativen Feuchtigkeitswerten $\varphi$.

**3.** Verfahren zum Charakterisieren nach Anspruch 2, wobei der Berichtigungsparameter ein Bezugswert $\tilde{S}_k^{ref}\big|_{\varphi 2}$ ist, der repräsentativ für das Messsignal $S_k(t_i)$ der ersten gasförmigen Probe für die gemessene relative Feuchtigkeit $\varphi 2$ ist.

**4.** Verfahren zum Charakterisieren nach Anspruch 2 oder 3, wobei:

∘ der Schritt des Bestimmens (140) des nützlichen Signals die folgenden Teilschritte beinhaltet:

• Bestimmen (141) eines Bezugswerts $\tilde{S}_k^{ref}\big|_{\varphi 1}$ repräsentativ für das Messsignal der ersten gasförmigen Probe für die gemessene relative Feuchtigkeit $\varphi 1$ aus der Kalibrierfunktion $h_k$;

• Bestimmen (142) eines nützlichen Signals $(Su_k(t_i \in P_1)$, das der ersten Probe zugeordnet ist, durch Subtrahieren vom Messsignal $(S_k(t_i \in P_1)$, das in der anfänglichen Phase P1 des bestimmten Bezugswerts $\tilde{S}_k^{ref}\big|_{\varphi 1}$ bestimmt wird;

• Bestimmen (143) eines Bezugswerts $Su_k^{ref}$ aus dem bestimmten nützlichen Signal $Su_k(t_i \in P_1)$, das der ersten Probe zugeordnet ist;

• Bestimmen (144) eines berichtigten nützlichen Signals $Suc_k(t_i \in P_2)$, das der zweiten gasförmigen Probe zugeordnet ist, durch Subtrahieren vom nützlichen Signal $Su_k(t_i \in P_2)$ des bestimmten Bezugswerts $Su_k^{ref}$;

o danach Charakterisieren (150) der Verbindungen von Interesse aus dem berichtigten nützlichen Signal $Suc_k(t_i \in P_2)$, das der zweiten gasförmigen Probe zugeordnet ist.

**5.** Verfahren zum Charakterisieren nach Anspruch 1, welches eine Phase (20) zum Bestimmen der notierten Kalibrierfunktion $f_k$ beinhaltet, die folgenden Schritte beinhaltend:

o Injizieren (21) der ersten gasförmigen Probe in die Messkammer, sodass mehrere Injektionszyklen ausgeführt werden, wobei jeder Zyklus eine erste Injektion der ersten gasförmigen Probe mit einer relativen Feuchtigkeit $\varphi 1$, gefolgt von einer zweiten Injektion der ersten gasförmigen Probe mit einer relativen Feuchtigkeit $\varphi 2$, die sich von $\varphi 1$ unterscheidet, beinhaltet, und Bestimmen (23) der Differenz der relativen Feuchtigkeit $\Delta \varphi$ zwischen $\varphi 1$ und $\varphi 2$, welche von einem Zyklus zum anderen variiert;

o Bestimmen (22) eines Messsignals $\widetilde{S_k}(t_i)$ im Laufe der verschiedenen Injektionszyklen, und Bestimmen einer Differenz von Bezugswerten $\Delta \tilde{S}_k^{ref}$ für jeden Injektionszyklus und für jede Differenz einer bestimmten relativen Fruchtigkeit $\Delta \varphi$ aus dem bestimmten Messsignal $\widetilde{S_k}(t_i)$ im Laufe der verschiedenen Injektionszyklen;

o Bestimmen (24) der Kalibrierfunktion $f_k$, die die Variation der Differenz eines Bezugswerts $\Delta \tilde{S}_k^{ref}$ in Abhängigkeit von der Differenz der relativen Feuchtigkeit $\Delta \varphi$ ausdrückt, aus den bestimmten Werten der Differenz eines Bezugswerts $\Delta \tilde{S}_k^{ref}$ und der bestimmten Werte der Differenz der relativen Feuchtigkeit $\Delta \varphi$.

**6.** Verfahren zum Charakterisieren nach Anspruch 5, wobei der Berichtigungsparameter die Summe ist aus:

o einem Wert der Differenz von Bezugswerten $\Delta \tilde{S}_k^{ref}$ für die Differenz der bestimmten relativen Feuchtigkeit $\Delta \varphi$ aus der relativen Feuchtigkeit, die in der ersten Phase P1 und der zweiten Phase P2 gemessen wird, und

o einem Bezugswert $Sk^{ref}\big|_{\varphi 1}$, der der ersten gasförmigen Probe zugeordnet wird, und aus dem

Messsignal ($S_k(t_i \in P_1)$) bestimmt wird, das in der ersten Phase P1 bestimmt wird.

7. Verfahren zum Charakterisieren nach Anspruch 6, wobei der Schritt zum Bestimmen (240) des nützlichen Signals die Subtraktion des Berichtigungsparameters von dem Messsignal ($S_k(t_i \in P_2)$) beinhaltet, das der zweiten gasförmigen Probe zugeordnet ist, und in der zweiten Phase P2 bestimmt wird.

8. Verfahren zum Charakterisieren nach einem der Ansprüche 1 bis 7, wobei die Kalibrierfunktion ($f_k$, $h_k$) eine polynominale, logarithmische oder exponentielle Funktion ist.

9. Verfahren zum Charakterisieren nach einem der Ansprüche 1 bis 8, wobei die Kalibrierfunktion ($f_k$, $h_k$) eine polynominale Funktion 2. Ordnung ist.

10. Verfahren zum Charakterisieren nach einem der Ansprüche 1 bis 9, wobei die elektronische Nase eine Vorrichtung zum Messen der Interaktionen zwischen den Verbindungen von Interesse und den Rezeptoren in der Art eines optischen Oberflächenplasmonenresonanz-Rezeptors beinhaltet.

11. Verfahren zum Charakterisieren nach einem der Ansprüche 1 bis 10, wobei die elektronische Nase eine Vorrichtung zum Messen der Interaktionen zwischen den Verbindungen von Interesse und den Rezeptoren in der Art von Widerstands-, piezoelektrischen, mechanischen, akustischen oder optischen Rezeptoren beinhaltet.

**Claims**

1. A method for characterising compounds of interest introduced into a measuring chamber (4) of an electronic nose including at least one sensitive site ($6_k$) having receptors with which the compounds of interest are able to interact by adsorption/desorption, the process including the following steps:

   ○ injecting, into the measuring chamber (4):

      • during a first phase P1, a first gas sample formed of a carrier gas without the compounds of interest, then
      • during a second phase P2, a second gas sample formed of at least the carrier gas and said compounds of interest;

   ○ determining, during said phases P1 and P2, a measurement signal ($S_k(t_i)$) representative of the interactions between the gas sample in presence and the receptors of the sensitive site ($6_k$), at different measurement time points $t_i$, in re-

sponse to an excitation signal emitted at the sensitive site ($6_k$);
   ○ **characterised in that** it further includes the following steps:

      ○ measuring (120; 220):

         • a value $\varphi 1$ of the relative humidity $\varphi$ in the measuring chamber during the first phase P1, and
         • a value $\varphi 2$ of the relative humidity $\varphi$ during the second phase P2, $\varphi 2$ being different from $\varphi 1$;

      ○ determining (130; 230) a corrective parameter ($\tilde{S}_k^{ref}|_{\varphi 2}$; $\Delta \tilde{S}_k^{ref}|_{\Delta \varphi}$) associated with the sensitive site ($6_k$), from:

         • at least the measured value $\varphi 2$ of the relative humidity, and
         • a calibration function ($f_k$, $h_k$) predetermined and associated with the sensitive site ($6_k$), the calibration function ($f_k$, $h_k$) expressing a variation of a parameter ($\tilde{S}_k^{ref}$; $\Delta \tilde{S}_k^{ref}$) representative of the measurement signal associated with the first gas sample as a function of the relative humidity ($\varphi$, $\Delta\varphi$);

      ○ determining a useful signal ($Su_k(t_i \in P2)$) by correction of the measurement signal ($S_k(t_i \in P2)$) associated with the second gas sample from at least the determined corrective parameter ($\tilde{S}_k^{ref}|_{\varphi 2}$; $\Delta \tilde{S}_k^{ref}|_{\Delta \varphi}$) and characterising the compounds of interest from the useful signal ($Su_k(t_i \in P2)$).

2. The characterisation method according to claim 1, including a phase (10) of determining the calibration function denoted $h_k$ including the following steps:

   ○ injecting (11) the first gas sample into the measuring chamber so that the relative humidity $\varphi$ varies progressively, and measuring (13) the relative humidity $\varphi$;
   ○ determining (12) a measurement signal $\tilde{S}_k(t_i)$ during the injection step (11), then determining a reference value $\tilde{S}_k^{ref}$ from the determined measurement signal $\tilde{S}_k(t_i)$ and for each measured relative humidity value $\varphi$;
   ○ determining (14) a calibration function $h_k$ ex-

pressing the variation of the reference value $\tilde{S}_k^{ref}$ as a function of the relative humidity $\varphi$, from the determined reference values $\tilde{S}_k^{ref}$ and the measured relative humidity values $\varphi$.

3. The characterisation method according to claim 2, wherein the corrective parameter is a reference value $\tilde{S}_k^{ref}|_{\varphi2}$ representative of the measurement signal $\tilde{S}_k(t_i)$ of the first gas sample for the measured relative humidity $\varphi2$.

4. The characterisation method according to claim 2 or 3, wherein:

   ◦ the step of determining (140) the useful signal includes the following substeps:

      • determining (141) a reference value $\tilde{S}_k^{ref}|_{\varphi1}$ representative of the measurement signal of the first gas sample for the measured relative humidity $\varphi1$, from the calibration function $h_k$;
      • determining (142) a useful signal $Su_k(t_i \in P1)$ associated with the first sample by subtraction of the determined reference value $\tilde{S}_k^{ref}|_{\varphi1}$ from the measurement signal $S_k(t_i \in P1)$ determined during the initial phase P1;
      • determining (143) a reference value $Su_k^{ref}$ from the determined useful signal $Su_k(t_i \in P1)$ associated with the first sample;
      • determining (144) a corrected useful signal $Suc_k(t_i \in P2)$ associated with the second gas sample by subtraction of the determined reference value $Su_k^{ref}$ from the useful signal $Su_k(t_i \in P2)$;

   ◦ then, characterising (150) the compounds of interest from the corrected useful signal $Suc_k(t_i \in P2)$ associated with the second gas sample.

5. The characterisation method according to claim 1, including a phase (20) of determining the calibration function denoted $f_k$ including the following steps:

   ◦ injecting (21) the first gas sample into the measuring chamber so that several injection cycles are performed, each cycle including a first injection of the first gas sample at a relative humidity $\varphi1$ followed by a second injection of the first gas sample at a relative humidity $\varphi2$ different from $\varphi1$, and determining (23) the relative humidity difference $\Delta\varphi$ between $\varphi1$ and $\varphi2$ varying from one cycle to another;
   ◦ determining (22) a measurement signal $\tilde{S}_k(t_i)$ throughout the different injection cycles, and determining a reference value difference $\Delta\tilde{S}_k^{ref}$ for each injection cycle and for each determined relative humidity difference $\Delta\varphi$, from of the measurement signal $\tilde{S}_k(t_i)$ determined throughout the different injection cycles;
   ◦ determining (24) the calibration function $f_k$ expressing the variation of the reference value difference $\Delta\tilde{S}_k^{ref}$ as a function of the relative humidity difference $\Delta\varphi$, from the determined values of the reference value difference $\Delta\tilde{S}_k^{ref}$ and the determined values of the relative humidity difference $\Delta\varphi$.

6. The characterisation method according to claim 5, wherein the corrective parameter is the sum of:

   ◦ a value of the reference value difference $\Delta\tilde{S}_k^{ref}$ for the relative humidity difference $\Delta\varphi$ determined from the relative humidity measured during the first phase P1 and the second phase P2, and

   ◦ a reference value $S_k^{ref}|_{\varphi1}$ associated with the first gas sample and determined from the measurement signal $S_k(t_i \in P1)$ determined during the first phase P1.

7. The characterisation method according to claim 6, wherein the step of determining (240) the useful signal includes the subtraction of the corrective parameter from the measurement signal $S_k(t_i \in P2)$ associated with the second gas sample and determined during the second phase P2.

8. The characterisation method according to any one of claims 1 to 7, wherein the calibration function ($f_k$; $h_k$) is a polynomial, logarithmic, or exponential function.

9. The characterisation method according to any one of claims 1 to 8, wherein the calibration function ($f_k$; $h_k$) is a 2-order polynomial function.

10. The characterisation method according to any one of claims 1 to 9, wherein the electronic nose includes a device for measuring the interactions between the compounds of interest and the surface plasmon resonance optical type receptors.

11. The characterisation method according to any one of claims 1 to 10, wherein the electronic nose includes a device for measuring the interactions between the compounds of interest and the resistive, piezoelectric, mechanical, acoustic or optical type receptors.

**Fig.1A**

**Fig.1B**

**Fig.1C**

**Fig.2**

**Fig.3A**

**Fig.3B**

**Fig.4**

**Fig.5**

**Fig.6**

$\tilde{S}_k(t_i) \rightarrow \Delta\tilde{S}_k^{ref}$

21 → 22

$\varphi_1 ; \varphi_2 \rightarrow \Delta\varphi$

23

$f_k / \Delta\tilde{S}_k^{ref} = f_k(\Delta\varphi)$

24

20

$S_k(t_i)$

200 → 210

$\varphi_1 ; \varphi_2 \rightarrow \Delta\varphi$

220

$S_k^{ref}|_{\varphi1} ; \Delta\tilde{S}_k^{ref}|_{\Delta\varphi}$

230

$Su_k(t \in P2) = S_k(t \in P2) - ( S_k^{ref}|_{\varphi1} + \Delta\tilde{S}_k^{ref}|_{\Delta\varphi} )$

240

250

**Fig.7**

Fig.8A

Fig.8B

Fig.8C

## RÉFÉRENCES CITÉES DANS LA DESCRIPTION

**Documents brevets cités dans la description**

- WO 2018158458 A

**Littérature non-brevet citée dans la description**

- **BRENET et al.** Highly-Selective Optoelectronic Nose based on Surface Plasmon résonance Imaging for Sensing Gas Phase Volatile Organic Compounds. *Anal. Chem.,* 2018, vol. 90 (16), 9879-9887

- **SHAO et al.** Mechanism and Characteristics of humidity Sensing with Polyvinyl Alcohol-Coated Fiber Surface Plasmon résonance Sensor. *Sensors,* 2018, vol. 18, 2029